# EUROPEAN PATENT SPECIFICATION

(11) **EP 3 976 830 B1**
(45) Date of publication and mention of the grant of the patent: **21.05.2025**
(21) Application number: 20728086.8
(22) Date of filing: 29.05.2020
(51) Int. Cl.: C12Q 1/6886

(54) **DETECTION OF HYPERMETHYLATED GENES FOR DIAGNOSING PANCREATIC CANCER**
NACHWEIS VON HYPERMETHYLIERTEN GENEN ZUR DIAGNOSE VON BAUCHSPEICHELDRÜSENKREBS
DÉTECTION DE GÈNES HYPERMÉTHYLÉS POUR LE DIAGNOSTIC DU CANCER DU PANCRÉAS

(30) Priority: 29.05.2019 EP 19305695
(43) Date of publication of application: 06.04.2022
(73) Proprietor: Université Paris Cité, 75006 Paris (FR); Centre national de la recherche scientifique, 75016 Paris (FR); Institut National de la Santé et de la Recherche Médicale, 75013 Paris (FR); ASSISTANCE PUBLIQUE - HÔPITAUX DE PARIS, 75004 Paris (FR)
(72) Inventor: WANG-RENAULT, Shufang, 92340 Bourg la Reine (FR); TALY, Valérie, 92340 Bourg la Reine (FR); BACHET, Jean, 92130 Issy Les Moulineaux (FR); LAURENT-PUIG, Pierre, 92190 Meudon (FR); PIETRASZ, Daniel, 75013 Paris (FR)
(74) Representative: Cabinet Becker et Associés
(86) International application number: PCT/EP2020/065099
(87) International publication number: WO 2020/240031

(56) References cited:
- EP-A1- 3 239 302
- WO-A1-2017/158158
- CA-A1- 3 023 335
- US-A1- 2018 258 498
- SHIRAISHI M ET AL: "A comprehensive catalog of CpG islands methylated in human lung adenocarcinomas for the identification of tumor suppressor genes", ONCOGENE, NATURE PUBLISHING GROUP UK, LONDON, vol. 21, no. 23, 23 May 2002 (2002-05-23), pages 3804 - 3813, XP002969561, ISSN: 0950-9232, DOI: 10.1038/SJ.ONC.1205454
- A. VINCENT ET AL: "Genome-Wide Analysis of Promoter Methylation Associated with Gene Expression Profile in Pancreatic Adenocarcinoma", CLINICAL CANCER RESEARCH, vol. 17, no. 13, 24 May 2011 (2011-05-24), pages 4341 - 4354, XP055092899, ISSN: 1078-0432, DOI: 10.1158/1078-0432.CCR-10-3431

## Description

### FILED OF THE INVENTION

The present invention relates to the field of oncology, more particularly the early diagnostic of pancreatic cancer. Thus, the present invention relates to a method for diagnosing or identifying pancreas cancer in a subject. Further, it can be used for monitoring the evolution of pancreas cancer in a subject being diagnosed for pancreas cancer and/or determining, adapting a suitable therapeutic regimen for said subject. The present invention also relates to a kit comprising primers and/or probes to detect, determine, identify hypermethylated genes.

### BACKGROUND OF THE INVENTION

Pancreas cancer, also known as pancreatic cancer, is a cancer developing from cells in the pancreas and starting to grow out of control. The pancreas has 2 main types of cells, exocrine cells and endocrine cells. It is very important to know if the cancer in the pancreas is an exocrine or endocrine cancer because exocrine cells and endocrine cells of the pancreas form different types of tumors. Therefore, they have distinct risk factors and causes, have different signs and symptoms, are diagnosed with different tests, are treated in different ways, and have different outlooks. Exocrine cancers are by far the most common type of pancreas cancers. About 95% of cancers of the exocrine pancreas are pancreatic adenocarcinomas. These cancers usually start in the ducts of the pancreas. Less often, they develop from the cells that make the pancreatic enzymes, in which case they are called acinar cell carcinomas. Other, less common exocrine cancers include adenosquamous carcinomas, squamous cell carcinomas, signet ring cell carcinomas, undifferentiated carcinomas, and undifferentiated carcinomas with giant cells.

Tumors of the endocrine pancreas are uncommon, making up less than 5% of all pancreatic cancers. As a group, they are often called pancreatic neuroendocrine tumors (NETs) or islet cell tumors. Pancreatic NETs can be benign or malignant.

Early symptoms of exocrine pancreatic cancer may include jaundice and related symptoms such as dark urine, light-colored or greasy stools, itchy skin; belly or back pain, weight loss and poor appetite, nausea and vomiting, gallbladder or liver enlargement, blood clots, fatty tissue abnormalities, diabetes. As discussed previously, symptoms of endocrine pancreatic cancer are different than exocrine pancreatic cancer and may include gastrinomas, glucagonomas, insulinomas, somatostatinomas.

Many risk factors can be listed, such as tobacco, overweight and obesity, exposure to certain chemicals used, age, gender, race, family history, genetic, diabetes, chronic pancreatitis, cirrhosis of the liver, stomach problems.

Pancreatic adenocarcinomas are the major cause of cancer mortality in Western societies. For example, in France, the incidence increases each year (12000 cases/year) and it will constitute in 2020 the second cause of cancer mortality in Western countries. [Bouvier et al. 2014] Life expectancy at 5 years, all stages together, is barely superior at 5%. [Coleman et al. 2003 & Eheman et al. 2012].

For more than 10 years, gemcitabine monotherapy has been the gold standard for advanced pancreatic adenocarcinoma. [Burris et al. 1997] Recently, two randomized phase III studies have demonstrated that the FOLFIRINOX protocol on one hand, and the combination of gemcitabine plus nab-paclitaxel on the other hand, were superior to gemcitabine monotherapy for objective response rate, progression-free survival and overall survival. [Conroy T et al. 2011 & Von Hoff DD et al. 2013] Despite these advances, adenocarcinomas of the pancreas remain particularly radio-resistant and chemo-resistant and the overall prognosis of all stages of patients has been little improved. Combined treatments and targeted therapies are being developed to improve reference treatments. The targeting of genes involved in tumor progression is a way of choice for improving treatments.

As discussed previously, ductal adenocarcinomas are the most frequent (80% of exocrine cancers) and by far the most problematic because the diagnosis is really difficult to establish due to a lack of early and specific clinical signs. At diagnosis, only 10% of patients can benefit from a curative surgical resection, 30 to 40% have a locally advanced disease against surgery and 50% to 60% of patients have distant metastases. [Siegel et al. 2015]. Mutations have been reported in adenocarcinoma of the pancreas. [Bardeesy et al. 2002] The predominant abnormalities concern the KRAS, TP53 or SMAD4 gene. The activation of the pro-oncogene KRAS appears early and is found in 80 to 90% of pancreatic cancers. [Almoguera et al. 1988 & Tada et al. 1993 & Morris JPt et al. 2010 & Kanda M et al. 2012] Mutations in the TP53 tumor suppressor gene are found in 50 to 75% of pancreatic cancers. [Hruban et al. 2008] TP53 mutations occur late in the pancreatic cancer progression and play a role in accelerating carcinogenesis. [Moore et al. 2003] SMAD4/DPC4 is also a tumor suppressor gene who are inactivated in 48 to 55% of pancreatic cancers. The loss of function of SMAD4 occurs late in pancreatic oncogenesis. But, other researches are needed to better understand and determine the prognosis or predictive value for SMAD4 mutation in pancreatic adenocarcinoma. Carbohydrate antigen 19-9 also called CA 19-9 is a serologic marker, which is highly prognostic in pancreatic adenocarcinoma. [Poruk et al. 2013] Cell free circulating tumor DNA (ctDNA) has also been evaluated to improve CA 19-9 value in the diagnostic of pancreatic cancer. Thus, Dabritz and colleagues were able to diagnose pancreatic adenocarcinoma with a sensitivity of 91% [Dabritz et al. 2009 & Pietrasz et al. 2017] 2017]. Vincent A. et al. ( CLINICAL CANCER RESEARCH, vol. 17, no. 13, 24 May 2011, pages 4341-4354) present further candidate methylation marker genes for the diagnosis of pancreas cancer.

Further, the identification of prognostic and/or predictive biomarkers could ultimately make it possible to better define the therapeutic strategy for a given patient (surgery and/or radiotherapy in particular), to find new therapeutic targets and to individualize treatments according to tumor molecular characteristics.

Currently, diagnosis of pancreatic cancer is usually done by biopsy during endoscopy or by inserting a needle through your skin and into your pancreas, by blood test to detect tumor markers, such as CA19-9 test, by imaging tests such as computerized tomography (CT) scans, magnetic resonance imaging (MRI) and, sometimes, positron emission tomography (PET) scans or endoscopic ultrasound (EUS). However, these methods require expensive medical equipment that generates considerable costs.

In this context, there is still a need to identify a sensitive and specific biomarker of pancreatic cancer, that can be used on body effluents, preferably blood samples by easy means for diagnosing, monitoring the evolution of cancer, determining a therapeutic regimen suitable for a subject diagnosed for pancreas cancer

The identification of candidate cancer biomarkers has exploded since the introduction of genome-wide sequencing of diseased tissue DNA using next-generation sequencing (NGS). However, such identification is useful for the early detection of cancer only if it can be done in the periphery, noninvasively. Unlike a mutation, which is a specific change in the DNA sequence and hence can be recognized unambiguously, DNA methylation is often present in multiple sites and has a spectrum of quantitative differences between tumor and normal tissues.

Methylation of the promoter and the first exon of tumor suppressor genes resulting in downregulation of gene expression has been shown to play a crucial role in tumorigenesis. Aberrant DNA methylation often occurs in the early stage in carcinogenesis, thus providing attractive potential markers for the early detection of cancer. For developing biomarkers for cancer screening and early detection, the increased methylation (hypermethylation) provides a positive readout with clear target regions for sensitive and specific assay development, and thus is advantageous over global hypomethylation for a clinical test.

Despite these attractive promises, challenges exist for translating methylated DNA marker candidates into useful clinical diagnosis markers. Several reviews have discussed the obstacles limiting the routine use of DNA methylation biomarkers in cancer therapy [Mikeska T, 2012; Delpu Y et al, 2013].

These reviews highlight that analytical sensitivity is a critical parameter for diagnostic applicability of methylation screening technology and that a good analytical sensitivity can be achieved only by combining several markers or by complementing pre-existing screening tests, because a single-marker approach is unlikely to have a sufficient sensitivity (>90%) for screening, except in extremely well-defined high-risk groups where the marker is linked with the etiology of cancer.

Another critical factor for a screening test is high specificity. Low specificity results in high numbers of false-positive results, which may lead to mental stress for patients and to unnecessary medical procedures, undermining the credibility and acceptance of the test in the population. The local circumstances in a methylation event require an assay to be quantitative enough to distinguish the truly cancer-related hypermethylation event from baseline methylation. It has also been reported that DNA methylation biomarkers are often not specific to one type of cancer but mostly conserved among tumor types. Thus, it seems challenging to propose a single DNA methylation alteration as a biomarker for a certain type of cancer, what is nevertheless ideal in order to treat the patient with an appropriate treatment as early as possible. Combination of several biomarkers has often been proposed to compensate for this lack of specificity, yet it also lowers the sensitivity of the test.

Altogether, the above-mentioned reviews show that, although proof of principle is already available for the great potential of methylation biomarkers in clinical use, the field faces many challenges for identifying ideal biomarkers exhibiting both a high sensitivity and a high specificity.

Moreover, none of these reviews discloses any study highlighting a methylation biomarker for diagnosing pancreatic cancer. This suggests that it is quite difficult to identify a sensitive and specific methylation biomarker of pancreas cancer.

In this scientific context, the inventors however observed, as disclosed in the experimental part below, that it is possible to use in pancreatic cancer, a combination of two specific methylation biomarkers, for diagnosing, and monitoring pancreatic cancer with an excellent sensitivity and specificity. These findings contrast with all the drawbacks suggested by the specialists in the field in the reviews cited above.

### SUMMARY OF THE INVENTION

The invention is defined by the claims. Any subject-matter falling outside the scope of the claims is provided for information purposes only.

The present invention relates to a method for diagnosing or identifying a pancreatic cancer in a subject, through the detection of the abnormal hypermethylation levels of specific genes in a biological sample of said subject. The inventors surprisingly identify two DNA methylation biomarkers that, alone, preferably in combination, can help diagnosing pancreas cancer or following-up the evolution of pancreas cancer in patients very specifically, discriminating with other types of cancers. They propose to measure the DNA hypermethylation of said genes by dPCR, in a body effluent sample, preferably a blood sample of the subject. The present invention also relates to kits and other tools for diagnosing pancreatic cancers.

Thus, in a first aspect, the invention relates to an *in vitro* method for diagnosing or identifying pancreas cancer in a subject, said method comprising determining the level or amount of methylation of the *POU4F1* gene and/or *HOXD8* gene in a biological sample of said subject.

In a second aspect, the invention relates to an *in vitro* method for monitoring the evolution of pancreas cancer in a subject being diagnosed for pancreas cancer, said method comprising:
a) determining the level or amount of methylation of at least one gene selected from the group consisting of the *POU4F1* gene and the *HOXD8* gene in a biological sample of said subject, at a first time point,
b) determining the level or amount of methylation of said at least one gene selected previously in the step a), in a biological sample of said subject, at a second time point, and
c) comparing the level or amount of methylation determined in step b) to the level or amount determined in step a) or to a reference value.

In a third aspect, the invention relates to a method for determining or adapting a therapeutic regimen suitable for a subject diagnosed for pancreas cancer comprising the steps of:
a) determining the level or amount of methylation of *POU4F1* gene and/or *HOXD8* gene in a biological sample of the subject prior to administration of treatment or during treatment of said subject,
b) determining the level or amount of methylation of *POU4F1* gene and/or *HOXD8* gene in a biological sample of the subject after administration of treatment of said subject,
c) comparing the level or amount of methylation determined in step b) to the level or amount of methylation determined in step a), or to a reference value,
d) adapting/modifying the therapeutic regimen for the subject based on the comparison of step c).

In another aspect, the invention relates to a method for predicting a clinical outcome in a subject afflicted with pancreas cancer comprising the following steps of:
a) determining the level or amount of methylation of *POU4F1* gene and/or *HOXD8* gene in a biological sample of said patient, and comparing said level or amount to a reference value,
b) predicting the clinical outcome based on the comparison of step a).

In another aspect, the invention relates to a kit comprising primers and/or probes targeting specifically the nucleotide region of SEQ ID NO:1 in the *POU4F1* gene or the nucleotide region of SEQ ID NO:3 in the *HOXD8* gene, said kit comprising preferably the primers of SEQ ID NO:5 and SEQ ID NO:6 and/or SEQ ID NO:7 and SEQ ID NO:8 and/or the probes of SEQ ID NO:9 and/or SEQ ID NO:10.

In another aspect, the invention relates to a microarray carrying nucleotides targeting specifically the nucleotide region of SEQ ID NO:1 in the *POU4F1* gene or the nucleotide region of SEQ ID NO:3 in the *HOXD8* gene, said microarray containing preferably the primers of SEQ ID NO:5 and/or SEQ ID NO:6 and/or SEQ ID NO:7 and/or SEQ ID NO:8

In a further aspect, the invention relates to a use of the kit or of the microarray for:
a) diagnosing or identifying pancreas cancer in a subject,
b) predicting a clinical outcome in a subject afflicted with pancreas cancer,
c) determining the therapeutic regimen of a subject with pancreas cancer, and/or
d) monitoring the progress of pancreas cancer in a subject being diagnosed for pancreas cancer.

The invention is particularly suited to detect and determine the hypermethylation of *POU4F1* gene, *HOXD8* gene, alone, preferably in combination, for helping to diagnose pancreas cancer at early stage very specifically and discriminating with other types of cancers or following-up the evolution of pancreas cancer patients previously diagnosed.

### LEGEND OF DRAWINGS

**Figure 1** discloses the methylation level of selected biomarkers *HOXD8* and *POU4F1* in tumor (n=20) and adjacent non-tumor tissue DNA (n= 18) from pancreas cancer patients by ddPCR. Mann-whitney test was used for the analysis of the hypermethylation difference between normal and adjacent tissue DNA.
**Figure 2** discloses the comparison between analyses of ctDNA by mutation detection using BPER NGS (MUT_SEQ) or methylation analysis by the two candidate genes analysis (METH_POS). The frequencies of mutated sequences as observed by BPER-NGS (Seq_Max, ordinates) and methylated sequences (ratio, abscises) are indicated.
**Figure 3** discloses the survival probability of patients according to their methylation status. (Validation cohort) METH_POS=0 correspond to patients negative for the methylated DNA and METH_POS=1 to the positive patients.
**Figure 4****.** PRODIGE 35 cohort: Overall and progression free survivals according to the Met-DNA status.
**Figure 5****.** Overall survivals according to the treatment arm and Met-DNA status. Panel a: PRODIGE 35 phase II trial results; Panel a1: PRODIGE 35 patients with Met-DNA Neg status; Panel a2: PRODIGE 35 patients with Met-DNA POS status.

### DETAILED DESCRIPTION OF THE INVENTION

### Definitions

As intended herein, the term "comprising" has the meaning of "including" or "containing", which means that when an object "comprises" one or several elements, other elements than those mentioned may also be included in the object. In contrast, when an object is said to "consist of" one or several elements, the object cannot include other elements than those mentioned.

According to the invention, the terms "subject", "individual", and "patient" are used interchangeably herein and refer to a mammal that may be healthy (without any symptoms of pancreatic cancer), thought to develop pancreatic cancer, is suspected of suffering from pancreatic cancer or suffered from pancreatic cancer. Said subject for example presents at least one of the following symptoms: jaundice and related symptoms such as dark urine, light-colored or greasy stools, itchy skin; belly or back pain, weight loss and poor appetite, nausea and vomiting, gallbladder or liver enlargement, blood clots, fatty tissue abnormalities, diabetes. Said subject may also have suffered from a pancreatic cancer in the past, has been treated, and is monitored for potential disease recurrence. Said subject may also seem to be healthy but is predisposed to develop a pancreatic cancer, for example tobacco, overweight obesity, genetic or because a member of his family is suffering or has suffered from the same disease. Subjects are preferably humans.

Typically, said diagnostic and monitoring methods involve the use of biological samples obtained from the patient. As used herein the term "biological sample" encompasses a variety of sample types obtained from a subject, that can be used in a diagnostic or monitoring assay. Biological samples include but are not limited to, blood and other liquid samples of biological origin such as body effluent or solid tissue samples such as a biopsy specimen. For example, biological samples include body effluent collected from an individual suspected of having a pancreatic cancer such as urine, pancreatic juice, feces. Therefore, biological samples encompass clinical samples, urine, pancreatic juice, feces, stools, blood sample, plasma and tissue samples, preferably blood sample.

In the field of cancer biomarkers, the most widely studied epigenetic modification is cytosine methylation. DNA methylation in the human genome occurs mostly at the cytosine residues in a CpG dinucleotide at the carbon-5 position, resulting in 5-methylcytosine. CpG dinucleotides are rare in the human genome (^{~}1%). CpG dinucleotides are often found to be in clusters of more than 200 bases with more than 50% of G+C content and a ratio of CpG frequencies of at least 0.6, which are known as CpG islands. Approximately 60% of the human gene promoters are associated with CpG islands. The CpG islands within the promoter regions are usually unmethylated in normal cells, except for some involved in tissue differentiation. In general, CpG island methylation is associated with transcriptional silencing. In cancer both global hypomethylation (decrease of overall DNA methylation) and localized hypermethylation, such as methylation of the promoter and the first exon of tumor suppressor genes, have been observed. DNA hypomethylation occurs at many genomic sequences, such as repetitive elements, retrotransposons, introns and similar elements, resulting in genomic instability, and can account for the activation of some proto-oncogenes and lead to loss of imprinting, as in the case of the *IGF2* gene (encoding IGF-2) in Wilms' tumor.

To our knowledge, no study has ever revealed any DNA methylation biomarker for diagnosing pancreatic cancer specifically.

Yet, the present inventors have observed that specific regions within the promoter, gene body or introns of two specific genes (namely *POU4F1 & HOXD8*) are abnormally hypermethylated in pancreatic cancer tumor samples specifically. Interestingly, these two genes had never been associated to pancreatic cancer.

The *"POU4F1* gene" of the invention designates a protein coding gene encoding POU domain, class 4, transcription factor 1 (*POU4F1*) also known as brain-specific homeobox/POU domain protein 3A (BRN3A), homeobox/POU domain protein RDC-1 or Oct-T1 protein. Its DNA sequence is located on chromosome 13 (78,598,362 - 78,603,560), more precisely on 13q31.1. Its DNA sequence is referred to as NC_000013.11. The present inventors have identified the DNA region preferably comprised between 79,176,072 - 79,177,072 (SEQ ID NO:1), more particularly between 79,176,472 - 79,176,672 (SEQ ID NO:2) to be significantly hypermethylated specifically in pancreatic tumor samples, by contrast with healthy samples and tumor samples of other cancers. They therefore propose to use this gene in a non-invasive, sensitive and reliable method for diagnosing or identifying pancreatic cancer in a subject.

The *"HOXD8* gene" of the invention designates a protein coding gene encoding Homeobox protein Hox-D8 (*HOXD8*)*.* Its DNA sequence is located on chromosome 2 (176,129,694 - 176,132,695), more precisely on 2q31.1. Its DNA sequence is referred to as NC_000002. The present inventors have identified the DNA region preferably comprised between 176,994,063-176,995,088 (SEQ ID NO:3) more particularly between 176,994,363 - 176,995,088 (SEQ ID NO:4) to be significantly hypermethylated specifically in pancreatic tumor samples, by contrast with healthy samples and tumor samples of other cancers. They therefore propose to use this gene in a non-invasive, sensitive and reliable method for diagnosing or identifying pancreatic cancer in a subject.

The inventors have shown that hypermethylation of these genes can be used as a sensitive and specific biomarker of pancreatic cancer in patients suffering thereof, even at early stage. The *POU4F1* gene, and *HOXD8* gene are hereafter referred to as the "biomarkers of the invention". They can be used individually, or in combination.

**Table 1. Useful sequence of interest in the POU4F1 gene and HOXD8 gene.**

| Region of interest | Nucleotide sequence |
|---|---|
| *POU4F1* | CGCCGCCGATGCCGCTGCCACCTGCCCGGCCGCCGCCGCCGCTGCCGCTGCC GCGCCGTGGTGCGCCGCCGCCGCCACCAGCCCGGGGTGCGGCAGCCCGGACGGC ATGTTCATGGCGGCCGCCGCCGCGGGGTGCGACAGGTGGCCCAGGCTGTGCATA TGCGGGTGAGGGTGCGCGGAGCCGCCCAGGAGCCCGCCGCCCGGGCCGCCGCC GCCGCCCCCCGGGCCGCCACCGCCGCCTCCCCCGGGGCCGCCGCCCGGGCCGCCG CCGCCGCCCGGGCCGCCACCGCCCCCCGGGCCGTCGTGGGCGCCGCCGCCGCCG GCCGCCGCGCCCGCGCCGCCCGCGCCGGCCATGAGCGCGAGCGACGGCGAGGA GATGTGGTCCAGCAGATCGCCGGGTTCGAGCGCCTGGTGGTGGTGGTGGTG GTGGTGGTGCGCCAGAGGCACCGTGGAAGTGGACGTGCACGGCACGCTGTTCAT CGTGTGGTACGTGGCGTCCGGCTTGAAAGGATGGCTCTTGCCCTGGGACACGGC GATGTCCACGGCCGCCAGCGCCTCGGCCCGCGCCAGCAGCGTCTCGTCCAGGCTG GCGAAGAGGTTGCTCTGCAGCTGCAGGCGACACAAACCAAACCAAAAAAACCAC AAAACCAAAAGAGCAAAACAAAACAACAGAAGAAACACACACACAGGCCGGAAA GCACAGCATGCGAAGGGCAAACACAAAGCAACCAAAATAACAACGGGTTTGGGG GCAGTGGAGAGCGGGAAAGACGGAGAGGGGGCACATTGACGACCAGGGAGGG GGCAGACGAGAAGGGATGGGAGCGTGGAGAGGGGGACAGAAGTAGGGAGAAA GGGGGACACAAGAACACATTCCGGAAACGGGCGTGGGAGACGAAAAAGAGGGA AAAGAAGAAATGGAAATGTAACTCGCAGCTGGGGACCCGTGTCACACACCCGAG CACGCACAGAGACTGCCTTTCTGAGGCGTGAAA (SEQ ID NO:1) |
| chr13: 79176072-79177072 | |
| *HOXD8* | ACCACTTCGATATGCCCCAACTCAAATGCACGGTCCGGTCCGTCAACACCTCTTGT CCACGTTCCCTGGGCTGCACCCGCGTGTCCAGAGCTGCAAAAGCCACGGGCAACC |
| chr2: 176994063-176995088 | TCTGCTTTTGCAGCCAGGGGCTCGGGGAGGCAGTCATTTGCTCCGCAGCCTCCTG GGAGTGGCCTCCTTGGCTCCCCCAAGTCTAAGGCTCCGCCGCGGCCCCTCCCTGCC GGCTGCGATCCGCATTCCCGCGGCCCCGGGGCACACGGAGCCCTTGGCAGTGCGT CTTTATGGGCCCCCTTTAAGGCCGGCGGAGGCATCTCGGGCCGGGCGCGGCGCTC CGTCCGTCGGCCGTAGCGACTGAACTGCGCGCGGATCCCTCCGCGGGGCTCCTCG TCCCCGTCACGCTGACTTTCCGTGCAGTGCTGTGGTGCGAAAATGCCTCGCCGGTG CGCACCGGGTCGGCAGCCTCGGCGGCGGGGGCGAGATTGGCGGGAGGGGGGC GCGGGGGGGGCGCGGTAAGAGGTGGCGGCGGGCAGAGGGTGTTTTTTTTCTTTT CCCTCCAGAGCCGGGGTTTGTAAACCGAGGCCAGAGTGTCCCCGTGGGCCGAGC GCACTTTTTTCTTGTCCGGGTGCGCTCAGTCACTGGTGCCTGAGAGGAAACAGTG GAGGCAGCGGGGCAGGTCGCCTGGGGCGTCGGCGATTATATTGCGGCCGAGCCG GGGCGCGCCGGGAAAGGCCGGGAGGGCGGCGGCGCGCGGGGGCTGGGCGAGG CCCCGCGACCCGCGAGGGAGGCGGCGCGAAGCCGAGGCGGCGGGCGCAAGAGC CGGGCATGAGCGCCCAGTAGCTGAGCGCCCGCGGCTGCCTGGCCTCAGAAGCGA CGCGCGAGCGCGGGCGGGCGGCAGCAGCGACGTAGCCCGGCGGTCCCGGCGGC GAGAGCAGCCGCCCCACAGGCCCCCGCGGCAGTGCGGCCGAGTCGAGGCTCGCT CTCTGGCTGCTTAGCGCCGCCCGCCCGCCCGGGGCCGCCGCCGCTGAC (SEQ ID NO:3) |
| *POU4F1* | GGTTCGAGCGCCTGGTGGTGGTGGTGGTGGTGGTGGTGCGCCAGAGGCACC GTGGAAGTGGACGTGCACGGCACGCTGTTCATCGTGTGGTACGTGGCGTCCGGCT TGAAAGGATGGCTCTTGCCCTGGGACACGGCGATGTCCACGGCCGCCAGCGCCTC GGCCCGCGCCAGCAGCGTCTCGTCCAGGCTGGCGAAG (SEQ ID NO:2) |
| chr13: 79176472-79176672 | |
| *HOXD8* | GGCGGAGGCATCTCGGGCCGGGCGCGGCGCTCCGTCCGTCGGCCGTAGCGACTG AACTGCGCGCGGATCCCTCCGCGGGGCTCCTCGTCCCCGTCACGCTGACTTTCCGT GCAGTGCTGTGGTGCGAAAATGCCTCGCCGGTGCGCACCGGGTCGGCAGCCTCG GCGGCGGGGGCGAGATTGGCGGGAGGGGGGCGCGGGGGGGGCGCGGTAAGA GGTGGCGGCGGGCAGAGGGTGTTTTTTTTCTTTTCCCTCCAGAGCCGGGGTTTGT AAACCGAGGCCAGAGTGTCCCCGTGGGCCGAGCGCACTTTTTTCTTGTCCGGGTG CGCTCAGTCACTGGTGCCTGAGAGGAAACAGTGGAGGCAGCGGGGCAGGTCGCC TGGGGCGTCGGCGATTATATTGCGGCCGAGCCGGGGCGCGCCGGGAAAGGCCG GGAGGGCGGCGGCGCGCGGGGGCTGGGCGAGGCCCCGCGACCCGCGAGGGAG GCGGCGCGAAGCCGAGGCGGCGGGCGCAAGAGCCGGGCATGAGCGCCCAGTAG CTGAGCGCCCGCGGCTGCCTGGCCTCAGAAGCGACGCGCGAGCGCGGGCGGGC GGCAGCAGCGACGTAGCCCGGCGGTCCCGGCGGCGAGAGCAGCCGCCCCACAG GCCCCCGCGGCAGTGCGGCCGAGTCGAGGCTCGCTCTCTGGCTGCTTAGCGCCGC CCGCCCGCCCGGGGCCGCCGCCGCTGAC (SEQ ID NO:4) |
| chr2: 176994363-176995088 | |

### Methods of the invention

In a first aspect, the invention relates to an *in vitro* method for diagnosing or identifying pancreas cancer in a subject, said method comprising determining the level or amount of methylation of the *POU4F1* gene or of the *HOXD8* gene in a biological sample of said subject.

In a preferred embodiment, said method comprises determining the level or amount of methylation in the nucleotide region of SEQ ID NO:1 in the *POU4F1* gene or in the nucleotide region of SEQ ID NO:3 in the *HOXD8* gene. More preferably, said method comprises determining the level or amount of methylation in the nucleotide region of SEQ ID NO:2 in the *POU4F1* gene or in the nucleotide region of SEQ ID NO:4 in the *HOXD8* gene.

In another embodiment, biological samples according to the invention are body effluents of said subject, for example, urine, pancreatic juice, stools, preferably a blood sample.

The method of the invention also comprises the step of comparing the level or amount of methylation of said gene(s) with the methylation level or amount of the same gene(s) that is determined in a reference sample. If the *POU4F1* gene and/or *HOXD8* gene are significantly hypermethylated in the biological sample of the tested subject as compared to the same biomarker in the reference sample, then the tested subject has a pancreatic cancer or has a high risk to have pancreatic cancer. The high risk of pancreatic cancer can be confirmed by biopsy or imaging tests.

Thus, in the method of the invention, if the said gene is hypermethylated as compared with a reference sample, then said subject is diagnosed or identified as suffering from a pancreatic cancer. In other words, a high level or amount of methylation is regarded as an indicator of pancreatic cancer, of invasive high-grade pancreatic cancer or of relapse of pancreatic cancer.

According to the present invention, the "reference sample" which is used to detect an "hypermethylation" for carrying out a diagnostic of pancreatic cancer or for following the evolution of pancreatic cancer is a biological sample. In particular it is a biological sample from a subject that does not suffer from pancreatic cancer or a biological sample from a subject who has been previously diagnosed as suffering from pancreatic cancer. Said biological sample can be composed of normal or healthy cells (or composed of both), tissue, a body fluid or a data set produced using information from a normal or healthy cell, tissue or body fluid. For example, said reference sample can be genomic DNA extracted from total blood or circulated DNA extracted from healthy subjects where said samples are the same as the one tested for the patients (for example plasma).

Preferably, it is a biological sample of a healthy subject that has no antecedent of nor predisposition to pancreatic cancer. Alternately, it is a biological sample of a subject who has been previously diagnosed as suffering from pancreatic cancer and the reference sample is a non-cancerous sample, such as a biological sample of a patient suffered from pancreatic cancer that is collected by biopsy near the tumor site (without tumor cells). More preferably, the control biological samples consist of a serie of samples from healthy subjects where the samples are the same as the ones tested for the patients (ie. Plasma or blood for example). More preferably, it is a blood or plasma sample obtained from a healthy subject. As used herein, the reference sample can be a previously collected sample of a subject suffering from pancreatic cancer that will be used to follow the evolution of pancreatic cancer from said subject by comparing it to the sample collected at time of evaluation.

In a more preferred embodiment, the reference sample according to the present invention is obtained in a healthy subject.

A number of techniques has been proposed to detect DNA methylation. The review of Delpu et al (2013) discloses some of them:
Methylation specific-PCR (MS-PCR) is a method in which two sets of PCR primers are specifically designed to amplify methylated and unmethylated DNA regions of interest. The detection of PCR products is originally performed by gel electrophoresis. This technique has been replaced by Quantitative MS-PCR (qMS-PCR), in which PCR amplification is monitored in real time by the incorporation of fluorescent molecules. This improvement allows for precise quantification of the DNA methylation levels of numerous specific regions and avoids the long electrophoresis step. Quantitative multiplex MS-PCR (QM-MS-PCR) and one step MS-PCR (OS-MS-PCR) are also available to co-amplify specific genes in tissues from different origins or to determine DNA methylation levels of a specific region without the DNA extraction procedure. qMS-PCR techniques are simple, rapid, inexpensive, highly-sensitive and easily standardized. They are currently one of the most commonly used techniques for cancer diagnosis in clinical use. Methylation-sensitive high-resolution melting (MS-HRM) is based on the fact that the nucleotide sequence of PCR products of bisulfite-treated DNA will differ depending on the methylation status of the DNA region of interest. The methylation level is determined by comparing the melting dissociation curves to standard PCR products of the same region containing known methylated CpG sites. COBRA, for combined bisulfite restriction analysis, uses the ability of bisulfite conversion to create new restriction enzyme sites or to maintain consensus sites of MSRE. After amplification, PCR products are digested with appropriate MSRE. The proportion of digested PCR products is compared to undigested PCR products by poly-acrylamide gel electrophoresis and image quantification software. This technique is reliably applied to DNA obtained from formalin-fixed paraffin embedded (FFPE) tissue sample. Moreover, this approach allows for the assessment of the DNA methylation of a large number of biological samples. More recently, high throughput approaches have been developed. For instance, Methyl Light is a high throughput quantitative methylation assay that uses fluorescent-based real time PCR (TaqMan^{®}, Applied Biosystems, Forster City, CA, USA) in combination to bisulfite treatment. Also combined with bisulfite treatment pyrosequencing is a quantitative DNA sequencing method in which light is emitted as a result of an enzymatic reaction representing each time a nucleotide is incorporated into the growing DNA chain. These quantitative techniques detect low amounts of methylated DNA in heterogeneous DNA preparation. Easily standardized, rapid and inexpensive, these techniques are increasingly used for clinical purpose.

More recently, next-generation sequencing (NGS) technologies significantly increased the resolution level of DNA methylation profiles. NGS can also be adapted to immuno-precipitated DNA fragment (Methyl DNA Immuno-precipitation sequencing also called MeDIP seq). Ultimately NGS permits the sequencing of the entire genome after bisulfite conversion. Beside these NGS approaches, high throughput single nucleotide polymorphism (SNP) genotyping systems are suitable for DNA methylation analysis from bisulfite-converted genomic DNA. Further, it is possible to use other methods well known to the skilled person, such as methods which directly analyze the unmodified DNA (for example nanopore sequencing), methods using specific restriction enzymes, methylated sequence enrichment methods (e.g. EpiMark methylated Enrichment kit, New England), immunoprecipitation methods.

All these methods for detecting DNA methylation are well-known in the art, for example qMS-PCR, MS-HRM, COBRA, MSRE, Methyl Light, NGS, SNP genotyping, pyrosequencing, microarray, ICE-cold PCR, and can be used for determining the methylation level or amount of the markers of the invention.

The method of the invention requires to detect the "level of methylation", "methylation level" or the "amount of methylation", depending on the detection technology which is used. According to the present invention, the terms "level of methylation" or "amount of methylation" refer to the determination of a quantitative measure. Thus, the terms "level of methylation" or "amount of methylation" can be used interchangeably.

An "hypermethylation" is determined for example if the methylation value (level or amount) of any of the biomarkers of the invention is significantly higher in the biological sample of the tested subject as compared with the methylation value of the corresponding biomarker measured in said reference sample. A significantly higher amount or level of methylation of at least one of the biomarkers of the invention in the biological sample of a subject as compared with the normal amount or level of methylation in the reference sample is an indication that the tested subject has a pancreatic cancer or has a high risk to have pancreatic cancer.

A "significantly higher amount or level of methylation" refers to a methylation amount or level that is greater than the standard error of the assay employed to assess said amount or level.

In a preferred embodiment of the invention, said methylation amount is determined by Next Generation Sequencing (NGS) or by qPCR, preferably by dPCR.

As used herein, the term "dPCR" stands for "digital PCR" and can be used interchangeably with the term "ddPCR" stands for "droplet digital PCR". It is a refinement of conventional PCR methods, wherein the sample is separated into a large number of partitions and the PCR reaction is carried out in each partition individually. This separation allows a more reliable collection and sensitive measurement of nucleic acid amounts. The method has been demonstrated as useful for studying variations in gene sequences - such as copy number variants and point mutations - and it is routinely used for clonal amplification of samples for next-generation sequencing. More precisely, the PCR solution is divided into smaller reactions through a water oil emulsion technique, which are then made to run PCR individually. For example, the PCR sample can be partitioned into pico to nanoliter-size samples and encapsulated into oil droplets. The oil droplets are made using a droplet generator that applies a vacuum to each of the wells. Depending of the system used, from 5-10 million picoliter droplets (25-50ul sample) to 20,000 oil nanoliter droplets (20 µL sample) can be created. Other type of compartments could also be used (such as microfabricated ones) as well as single tube limited dilutions.

In a more preferred embodiment, the level or amount of methylation of the *POU4F1* gene and/or of the *HOXD8* gene is determined by dPCR by using anyone of the primers targeting said nucleotide region of *POU4F1* gene of SEQ ID NO:1 or SEQ ID NO:2 or *HOXD8* gene of SEQ ID NO:3 or SEQ ID NO:4, more particularly the primers of SEQ ID NO:5, SEQ ID NO:6, SEQ ID NO:7 or SEQ ID NO:8 or combination of the primers SEQ ID NO:5 and SEQ ID NO:6 and/or combination of the primers SEQ ID NO:7 and SEQ ID NO:8.

In another preferred embodiment, the level or amount of methylation of the *POU4F1* gene and/or of the *HOXD8* gene is determined by dPCR by using anyone of the probes targeting said nucleotide region of *POU4F1* gene of SEQ ID NO:1 or SEQ ID NO:2 or *HOXD8* gene of SEQ ID NO:3 or SEQ ID NO:4, more particularly the probes of SEQ ID NO:9 and/or SEQ ID NO:10.

In another embodiment, the level or amount of methylation of the *POU4F1* gene, or of the *HOXD8* gene is determined by dPCR by using the primers of SEQ ID NO:5 and SEQ ID NO:6 and/or SEQ ID NO:7 and SEQ ID NO:8 and using the probes targeting said nucleotide region of *POU4F1* gene of SEQ ID NO:1 or SEQ ID NO:2 or *HOXD8* gene of SEQ ID NO:3 or SEQ ID NO:4, more particularly the probes of SEQ ID NO:9 and/or SEQ ID NO:10.

In a more preferred embodiment, the level or amount of methylation of the *POU4F1* gene, or of the *HOXD8* gene is determined by dPCR by using the primers of SEQ ID NO:5 and SEQ ID NO:6 and/or SEQ ID NO:7 and SEQ ID NO:8 and using the probes of SEQ ID NO:9 and/or SEQ ID NO:10

According to a preferred embodiment of the invention to enhance the sensitivity and/or the specificity of the diagnostic method, the inventors show that using and combining the two biomarkers of the invention, it is possible to significantly enhance the sensitivity and/or specificity of said method. Preferably, said method comprises determining simultaneously or sequentially the level or amount of methylation of the *POU4F1* gene and the *HOXD8* gene in a biological sample of said subject.

Exemplary primers that can be used for determining hypermethylation of the *POU4F1* gene are of SEQ ID NO:5 and/or SEQ ID NO:6. Exemplary primers that can be used for determining hypermethylation of the *HOXD8* gene are of SEQ ID NO:7 and/or SEQ ID NO:8.

The primers that can be used in this preferred embodiment are reflected on Table 2 below and in SEQ ID NO: 5-8.

**Table 2. Useful primers of interest according to the present invention for determining the biomarkers of the invention by dPCR.**

| Primers | Nucleotide sequence |
|---|---|
| *POU4F1* FP | TGGTGCGTTAGAGGTATCGT. **(SEQ ID NO:5)** |
| *POU4F1* RP | AAACCATCCTTTCAAACCGA. **(SEQ ID NO:6)** |
| *HOXD8* FP | AGAGTCGGGGTTTGTAAATCG. **(SEQ ID NO:7)** |
| *HOXD8* RP | CTCTCAAACACCAATAACTAAACG. **(SEQ ID NO:8)** |

In one exemplary embodiment, the present method can be performed by using gene expression assays (e.g., dPCR) that use fluorogenic probes to enable the detection of the PCR products that accumulate during PCR. Such assays are for example TaqMan^{®} gene expression assays using probes containing minor groove binding (MGB) moiety that enhances the Tₘ differential between matched and mismatched probes. In addition, these MGB probes may contain a non-fluorescent quencher (NFQ) that enhances spectral resolution when using multiple dyes in a reaction.

According to a preferred embodiment of the invention to enhance the sensitivity and/or the specificity of the diagnostic method, the inventors show that using probes targeting *POU4F1* gene and/or *HOXD8* gene, it is possible to significantly enhance the sensitivity and/or specificity of said method.

Exemplary probe that can be used for determining hypermethylation of the *POU4F1* gene is of SEQ ID NO:9. Exemplary probe that can be used for determining hypermethylation of the *HOXD8* gene is of SEQ ID NO:10.

Said probes that can be used in this preferred embodiment are reflected on Table 3 below and in SEQ ID NO: 9-10.

**Table 3. Useful probes of interest according to the present invention.**

| Probes | Nucleotide sequence |
|---|---|
| *POU4F1* | FAM- CAACGTACCGTACACGTCCA-MGB-NFQ. **(SEQ ID NO:9)** |
| *HOXD8* | FAM-TCGTGGGTCGAGCGTA-MGB-NFQ. **(SEQ ID NO:10)** |

In another aspect, the biomarkers of the invention can be used for monitoring *in vitro* the evolution of pancreas cancer in a subject being diagnosed for pancreas cancer, said method comprising:
a) determining the level or amount of methylation of at least one gene selected from the group consisting of the *POU4F1* gene and the *HOXD8* gene in a biological sample of said subject, at a first time point,
b) determining the level or amount of methylation of said at least one gene selected previously in the step a), in a biological sample of said subject, at a second time point, and
c) comparing the level or amount of methylation determined in step b) to the level or amount determined in step a) or to a reference value.

In a preferred embodiment of this aspect, the two biomarkers of the invention can be used together, simultaneously or sequentially to follow the evolution of pancreas cancer in a subject being diagnosed for pancreas cancer. Thus, the *POU4F1* gene and the *HOXD8* gene can be used to implement this aspect of the present invention.

It can be concluded that the malignancy of the pancreatic cancer is worsening if the level or amount of methylation determined in step b) is significantly higher than the level or amount determined in step a). In other words, the tested subject has a disease that evolves badly, even though the subject may be treated already.

If the subject is treated already, a first sample may have been taken from the subject prior to treatment for pancreatic cancer and a second sample may have been taken from the subject after being treated for pancreatic cancer. Consequently, said first time point is preferably prior to treatment of said subject and said second time point is after said subject has been treated for pancreatic cancer.

In this case, the method of the invention can be used for assessing the efficiency of said treatment.

In the context of the invention, if the methylation of the *HOXD8* gene or the *POU4F1* gene or a combination of these genes is/are significantly decreased in the biological sample acquired after the treatment has been administered as compared to the same biomarker(s) in a sample acquired before the treatment has been administered, then the tested subject is likely to be responding efficiently to the tested treatment (therefore being a "responder").

Conversely, if the methylation of the *HOXD8* gene or the *POU4F1* gene or a combination of these genes is/are significantly increased in the biological sample acquired after the treatment has been administered, as compared to the same biomarker(s) in a sample acquired before the treatment has been administered, then the tested subject is likely not to be responding efficiently to the tested treatment (therefore being a "non-responder").

As used herein, a "non-responder" is considered as a patient with a progressive disease or a stable disease as defined according to RECIST 1.1 criteria.

As used herein, "treatments" may include some combination of surgery, chemotherapy, radiation therapy and targeted therapy. In a preferred embodiment, the treatment is surgery.

In a particular embodiment, the sample in step a) is obtained prior to the treatment for pancreas cancer and the sample in step b) is obtained after said subject has been treated for pancreas cancer.

In another particular embodiment, the subject has been previously treated for pancreas cancer and the biomarker of the present invention can be used to evaluate the efficiency of the treatment. Therefore, the level or amount of methylation is determined only once to determine if the subject has a level or amount of methylation that has been reduced comparing to a previous level or amount of methylation determined prior to the treatment. In another aspect, the level or amount of methylation is determined only once to determine if the subject has a hypermethylation of *POU4F1* or *HOXD8* alone or in combination. If said subject has a decreasing level or amount of methylation, then the treatment is effective. Alternately, if said subject does not have hypermethylation, the subject no longer has pancreatic cancer and is treated.

In this case, according to a particular embodiment, the subject has been treated for pancreas cancer in step a) and the level or amount of methylation is determined only once in step b) after said subject has been treated in step a) for pancreas cancer. Therefore, the level or amount of methylation is determined only once in step b) and the determination of the level or amount of methylation is optional for a subject that has been previously diagnosed.

In a particular embodiment, the reference value is obtained in a healthy subject.

In another aspect, the biomarkers of the invention can be used in a method for determining or adapting a therapeutic regimen suitable for a subject diagnosed for pancreas cancer comprising the step of:
a) determining the level or amount of methylation of *POU4F1* gene and/or *HOXD8* gene in a biological sample of the subject prior to administration of treatment or during treatment of said subject,
b) determining the level or amount of methylation of *POU4F1* gene and/or *HOXD8* gene in a biological sample of the subject after administration of treatment of said subject,
c) comparing the level or amount of methylation determined in step b) to the level or amount of methylation determined in step a), or to a reference value,
d) adapting/modifying the therapeutic regimen for the subject based on the comparison of step c).

In a preferred embodiment of this aspect, the two biomarkers of the invention can be used together, simultaneously or sequentially to determine or adapt the therapeutic regimen for a subject diagnosed for pancreas cancer. Thus, the *POU4F1* gene and the *HOXD8* gene can be used to implement this aspect of the present invention.

In particular, said therapeutic regimen will have to be highly efficient if the level or amount of said biomarker(s) is significantly inferior to the level or amount of said biomarker(s) determined before said treatment.

In particular, said therapeutic regimen is to be changed if the level or amount of said biomarker(s) is still significantly superior to the level or amount of said biomarker(s) determined before said treatment

In another aspect, the biomarkers of the invention can be used to predict the outcome of pancreatic cancer patients. Also, they can be used to aid the skilled oncologist in the selection of appropriate treatments for maximizing the survival of the patients. Appropriate treatments are for example chemotherapeutic treatments, immunotherapeutic treatments, radiotherapeutic treatments and/or surgery. Specifically, said patients have been treated or will be treated with surgery.

Thus, the biomarkers of the invention can be used in a method for predicting a clinical outcome in a subject afflicted with pancreas cancer comprising the step of:
a) determining the level or amount of methylation of *POU4F1* gene and/or *HOXD8* gene in a biological sample of said patient, and comparing said level or amount to a reference value,
b) predicting the clinical outcome based on the comparison of step a).

If the *HOXD8* gene or the *POU4F1* gene or a combination of these genes is/are significantly hypermethylated in the biological sample of the tested subject as compared to the same biomarker in the reference sample, then the tested subject is likely to have a bad clinical outcome.

Conversely, if the *HOXD8* gene or the *POU4F1* gene or a combination of these genes present(s) comparable methylation level in the biological sample of the tested subject as compared to the same biomarker in a reference sample (from a healthy subject or a subject not suffering from a gastric cancer), then the tested subject is likely to have a good clinical outcome.

Conversely, if the *HOXD8* gene or the *POU4F1* gene or a combination of these genes is/are not significantly hypermethylated in the biological sample of the tested subject as compared to the same biomarker in a reference sample of the same subject, then the tested subject is likely to have a good clinical outcome.

### Kits of the invention

The present invention furthermore provides diagnostic, monitoring tools for determining the hypermethylation biomarkers of the invention in order to diagnose or monitor pancreatic cancer.

First, the present invention relates to nucleic acids which are useful to detect the hypermethylation regions highlighted above. Within the scope of the present invention, by "nucleic acid" is meant mRNA, genomic DNA or cDNA derived from mRNA. These nucleic acids are preferentially primers or probes.

In a preferred embodiment, the invention relates to a kit comprising primers targeting specifically the nucleotide region of SEQ ID NO:1, more preferably the nucleotide region of SEQ ID NO:2 in the *POU4F1* gene or the nucleotide region of SEQ ID NO:3, more preferably the nucleotide region of SEQ ID NO:4 in the *HOXD8* gene, said kit comprising preferably the primers of SEQ ID NO:5 or SEQ ID NO:6 or SEQ ID NO:7 or SEQ ID NO:8.

According to the preferred embodiment, said kit comprising more preferably combination of primers selecting in the group of SEQ ID NO:5, SEQ ID NO:6, SEQ ID NO:7, SEQ ID NO:8. Said combinations preferably used according to the invention are: SEQ ID NO:5 and SEQ ID NO:6, or SEQ ID NO:7 and SEQ ID NO:8.

In a particular embodiment, said kit comprises primers targeting specifically the nucleotide region of SEQ ID NO:1 in the *POU4F1* gene or the nucleotide region of SEQ ID NO:3 in the *HOXD8* gene, preferably the primers of SEQ ID NO:5 or SEQ ID NO:6 or SEQ ID NO:7 or SEQ ID NO:8

In another aspect the invention relates to a kit comprising probes targeting specifically the nucleotide region of SEQ ID NO:1, more preferably the nucleotide region of SEQ ID NO:2 in the *POU4F1* gene or the nucleotide region of SEQ ID NO:3, more preferably the nucleotide region of SEQ ID NO:4 in the *HOXD8* gene, said kit comprising preferably the probes of SEQ ID NO:9 and/or SEQ ID NO:10.

In another aspect the invention relates to a kit comprising primers and probes targeting specifically the nucleotide region of SEQ ID NO:1 in the *POU4F1* gene or the nucleotide region of SEQ ID NO:3 in the *HOXD8* gene. In a preferred embodiment, the kit comprising primers and probes targeting specifically the nucleotide region of SEQ ID NO:2 in the *POU4F1* gene or the nucleotide region of SEQ ID NO:4 in the *HOXD8* gene.

As used herein, the term "primers" designates isolated nucleic acid molecules that can specifically hybridize or anneal to 5' or 3' regions of a target genomic region (plus and minus strands, respectively, or vice-versa). In general, they are from about 10 to 30 nucleotides in length and anneal at both extremities of a region containing about 50 to 200 nucleotides in length. Under appropriate conditions and with appropriate reagents, such primers permit the amplification of a nucleic acid molecule comprising the nucleotide sequence flanked by the primers. Under other aspect of the invention, the primers can be used by pairs, they are often referred to as "primers pair" or "primers set".

As used herein, the term "probes" designates molecules that are capable of specifically hybridizing a genomic region of interest. They are useful to highlight the presence of said genomic region in biological samples. These probes may comprise at least one non-natural nucleotide, e.g., a peptide nucleic acid (PNA), a peptide nucleic acid having a phosphate group (PHONA), a bridged nucleic acid or locked nucleic acid (BNA or LNA), and a morpholino nucleic acid. Non-natural nucleotides also include chemically modified nucleic acids or nucleic acid analogs such as methylphosphonate-type DNA or RNA, phosphorothioate-type DNA or RNA, phosphoramidate-type DNA or RNA, and 2'-O-methyl-type DNA or RNA.

In a preferred embodiment, the probes of the invention comprise at least 15, consecutive nucleotides which are complementary of bisulfited or fragments thereof. In a more preferred embodiment, the molecules which can be used as a probe according to the present invention have a total minimum size of 15 nucleotides. In an even more preferred embodiment, these molecules comprise between 15 and 30 nucleotides (in total).

For certain uses, the probes and/or primers of the invention may be labeled - directly or indirectly - with a detectable label. Said label may be of any kind, depending on the experiment which is to be performed. Said label may be a radioactive isotope (such as ³²P, ³³P, ³⁵S, ³H or ¹²⁵I, or a nonradioactive entity which is selected from ligands (such as biotin, avidin or streptavidin), dioxygenin, haptens, colorants and luminescent agents (such as radioluminescent, chemiluminescent, bioluminescent, fluorescent or phosphorescent agents). Preferably, 6-carboxyfluorescein (FAM) and VIC are used. Non-labeled polynucleotide sequences may also be used, directly, as a probe or primer, for example in PCR-based processes (e.g., in quantitative PCR).

In a particular aspect, the probes of the invention are TaqMan probes of SEQ ID NO:9-10.

In another aspect, the invention relates to a microarray carrying nucleotides targeting specifically the nucleotide region of SEQ ID NO:1 in the *POU4F1* gene or the nucleotide region of SEQ ID NO:3 in the *HOXD8* gene, said microarray containing preferably the primers of SEQ ID NO:5 and/or SEQ ID NO:6 and/or SEQ ID NO:7 and/or SEQ ID NO:8 and/or SEQ ID NO:9 and/or SEQ ID NO:10. In a preferred embodiment, the microarray carrying nucleotides targeting specifically the nucleotide region of SEQ ID NO:2 in the *POU4F1* gene or the nucleotide region of SEQ ID NO:4 in the *HOXD8* gene, said microarray containing preferably the primers of SEQ ID NO:5 and/or SEQ ID NO:6 and/or SEQ ID NO:7 and/or SEQ ID NO:8 and/or SEQ ID NO:9 and/or SEQ ID NO:10.

These exemplary primers and probes are summarized in Tables 2 and 3 above.

The present invention also relates to a kit and/or microarray that can be used for:
a) diagnosing or identifying pancreas cancer in a subject,
b) predicting a clinical outcome in a subject afflicted with pancreas cancer,
c) determining or adapting the therapeutic regimen of a subject with pancreas cancer, and/or
d) monitoring the progress of pancreas cancer in a subject being diagnosed for pancreas cancer.

Further aspects and advantages of the invention will be disclosed in the following examples, which should be considered illustrative.

### EXAMPLES

### MATERIAL AND METHODS:

### 1. Identification of DNA methylation biomarkers and hypermethylation on tissue sample DNA and healthy plasma circulating cell free DNA (ccfDNA):

### 1.1 Patients and healthy control individuals.

Twenty pancreatic cancer patients were included. Matched tumor and adjacent non-tumor tissue biopsies were collected from each patient. The local ethics committee approved this study and informed written consent was obtained from all the patients.

### 1.2 Tumor sample preparation, storage, DNA extraction, and quantification

Tumor and adjacent non-tumor tissue biopsies were flash frozen in liquid nitrogen immediately after resection until further analysis. Each tumor was reviewed by a pathologist and the tumor cell content was assessed by hematoxylin-eosin-safran staining. DNA was extracted with the QlAampDNAMini Kit (Qiagen) according to the manufacturer's instructions. DNA concentration was measured by Qubit 2.0 fluorometer (Invitrogen, Life Technologies) with the use of the dsDNA BR Assay (Invitrogen). Extracted DNA samples were stored at -20 °C.

### 1.3 Plasma sample preparation, storage, DNA extraction, and quantification

Plasma samples of healthy individuals were received in dry ice, aliquoted and immediately frozen at - 80 °C. Before extraction, plasma samples were centrifuged at 3000g for 10 min and then extracted with the use of the QIAmp Circulating Nucleic Acid Kit (Qiagen) or Maxwell according to the manufacturer's instructions. The quantity of DNA was measured by Qubit 2.0 fluorometer (Invitrogen, Life Technologies) with the use of the dsDNA HS Assay (Invitrogen). Extracted DNA samples were stored at -20 °C before testing.

### 1.4 Selection of candidate biomarkers for pancreas cancer patients based on public methylation database TCGA.

Methylation data of pancreatic cancer patients from The Cancer Genome Atlas (TCGA) (http://cancergenome.nih.gov/) were analyzed with the use of a homemade R script (see above). A list of 485,577 CpGs annotated with genes including their methylation level in healthy and tumor tissue, the difference in methylation level between healthy and tumor tissue (the fold change), correlation with amount of tumor cells and normal cells in cancer patients, and a Wilcoxon test and statistics was generated. Based on this information, we selected 2 candidate genes (*HOXD8, POU4F1*) containing CpGs that were significantly differentially methylated between tumor and healthy pancreas tissues.

### 1.5 Tissue DNA, plasma ccfDNA bisulfite conversion

Tissue DNA or plasma DNA was modified by bisulfite using the EZ DNA Methylation-Gold Kit (Zymo Research). In brief, bisulfite reaction was carried out in a thermocycler at 98 °C for 12 min and 64 °C for 2h35 min. The cleanup of bisulfite-converted DNA followed the recommendations of the manufacturer and converted DNA was eluted in M-Elution Buffer and stored at -20 °C.

### 1.6 Detection of methylation changes of selected biomarkers by droplet-based digital PCR

The hypermethylation of selected biomarkers in tumor DNA was validated by ddPCR (Raindrop or QX-200 systems, BIO-RAD Technologies) as described before [Garrigou, S., et al. 2016]. Duplex format was used to analyze hypermethylation with albumin for normalizing the DNA amount. Primers and probes were listed in the table 2 and 3. In brief, 12.5µL Kapa probe Fast qPCR master mix (Kapa Biosystems) was mixed with the assay solution containing: 0.75µL 40 mM dNTP Mix (New England BioLabs), 0.5µL 25 mM MgCl2, 1µL 25x Droplet Stabilizer (RainDance Technologies), 1.25µL 20x Assay Mix containing 8µM of forward and reverse primers, 4µM of 6-FAM and 12µM of VIC Taqman^{®} labeled-probes, and target modified DNA template to a final reaction volume of 25µL. When possible, a minimum of 10ng of modified DNA was used in each reaction.

A limit of blank (LOB) has been calculated as described previously [Taly, V., et al. 2013]. It is defined by the frequency of positive droplets measured in normal control DNA samples with no hyper-methylated DNA present. The calculated LOB was subtracted from each sample for calculating their methylation level.

The sample analysis was performed following the procedure described earlier [Taly, V., et al. 2013. Samples were considered positive when the number of observed droplets was higher than LOB value. The methylation level of each sample was calculated as the ratio of the number of droplets containing methylated sequences over the number of droplets containing albumin sequences.

Two DNA controls were used for ensuring the proper realization of the modification treatment (Positive control: universal hypermethylated DNA and negative control: normal human genomic DNA).

### 1.7 Measurement of the methylation level of selected biomarkers in plasma circulating cell free DNA (ccfDNA) from healthy individuals

DNA methylation of selected biomarkers in plasma from healthy individuals was measured by ddPCR (Raindrop or QX-200 systems, BIO-RAD Technologies) with the use of same reaction conditions as described before. Duplex format was used to analyze hypermethylation with albumin for normalizing the DNA amount. The same primers and probes were used as listed in the table 2 and 3.

### 1.8 Calculation of detection sensitivity and specificity

The sum of the average and standard deviation of methylation level in non-tumor tissue DNA was used as the threshold for calculating sensitivity and specificity of each selected biomarker. Sensitivity is the percentage of the patients showing higher methylation level in tumor tissues than the threshold. Specificity is the percentage of the patients showing lower methylation level in non-tumor tissues than the threshold.

### 2. Validation in two cohorts

### 2.1 First cohort named "Validation cohort"

### 2.1.1 Patients plasma sample preparation, storage, DNA extraction, and quantification

From January 2011 to June 2018, plasmas of all consecutive patients with histologically proven metastatic PAC, receiving first chemotherapy protocol, were prospectively collected in our Oncological Department (n=100). Blood samples were collected just before the first cycle of chemotherapy. All the patients signed an informed consent form, approved by the ethic committee (CPP Ile-de-France 2014/59NICB). The following data were collected in a prospective database: clinical and pathological characteristics (gender, age, medical history, date of diagnosis, location of the primary tumor, primary tumor diameter, tumor differentiation grade, stage of the disease), follow-up data (date of primary resection, date and type of relapse, date of diagnosis of metastatic disease, date and type of chemotherapy regimen, date of death or last follow-up) and biologic data (CEA, CA 19-9, albuminemia, bilirubinemia).

Patient blood samples (9mL) were withdrawn from a central catheter and placed in EDTA tubes. The collected samples were centrifuged at 3,500 rpm for 15 min at 4°C within 3 hours of blood draw. Plasma was stored at -80°C until further use. DNA was extracted from plasma with QlAamp^{®} Circulating Nucleic Acid kit (Qiagen, Hilden, Germany) according to the manufacturer's instructions. Incubation with proteinase K was performed for 30 min at 68°C. Extracted DNA from 2mL of plasma was eluted with 50µL buffer AVE and stored at -80°C. DNA quantity was assessed using the Qubit^{™} dsDNA HS (High Sensitivity) Assay kit (Thermo Fisher).

### 2.1.2 Droplet-based digital PCR

All patients' plasma samples were screened for the 2 methylated selected genes by ddPCR using BIO-RAD ddpcr Quantasoft^{®} system (Biorad Technologies). Using this system, single target DNA molecules were compartmentalized in droplets together with validated fluorogenic TaqMan^{™} as described previously. Analyses were performed as described previously. after testing all samples using Quantasoft Software (version 1.7, Biorad Technologies) following standard procedures. Samples were considered as positive for ctDNA when presenting positivity for the two gene markers. Samples positive for only one marker gene were further analyzed by Next generation sequencing. Samples that were also positive for the presence of a cancer-related mutation by NGS were further considered as positive for ctDNA.

### 2.1.3 Next Generation Sequencing (NGS).

All patients' plasma samples were tested by NGS to correlate methylation status to mutation status. Sequencing libraries were prepared from circulating-free DNA using Ion AmpliSeq^{™} Colon and Lung Cancer Research Panel v2 (Thermo Fisher). According to manufacturer's protocols, 10ng of DNA for each sample was used as input for library preparation with the Ion AmpliSeq^{™} Library Kit 2.0 (Thermo Fisher). The pooled barcoded libraries (max 96) were processed on Ion Chef^{™} System using the Ion PI Hi-Q Chef Kit (A27198) and sequenced on the Ion Proton^{™} System using and Ion PI Chip Kit v3 (A26771). The NGS analysis method has been specifically developed to detect low allele frequency mutations, the sensitivity and specificity of which have been validated in positive and negative controls. Samples were analyzed using the BPER procedure that we developed (see Pecuchet et al. Clinical Chemistry 2016 & Pietrasz et al. Clinical Cancer Research 2017).

### 2.2 Validation in prospective cohort (Prodige 35)

The aim of this study was to assess the prognostic value of Methylated circulating tumor DNA (Met-DNA) in metastatic Pancreatic Adenocarcinoma (mPAC). Prognostic value of Met-DNA was assessed in a prospective cohort of mPAC (Validation cohort), correlated with NGS, then in one prospective independent validation cohorts from two randomized phase II trials (PRODIGE 35).

**PRODIGE 35 - PANOPTIMOX** : Patients randomized to receive either 6m FOLFIRINOX (arm A), 4m FOLFIRINOX followed by LV5FU2 maintenance treatment for controlled pts, and treatment reintroduction at disease progression (arm B), or a sequential treatment alternating gemcitabine and FOLFIRI.3 every 2m (arm C).

**Table 4. Details of the included cohorts.**

| **Variables** | | | **Validation Cohort N = 100** | **PRODIGE 35 N = 177** |
|---|---|---|---|---|
| **Gender** | | | | |
| | | **Male** | 61 (61%) | 110 (62.1%) |
| | | **Female** | 39 (39%) | 67 (37.9%) |
| **Age*, years** | | | 66.5 (32.0-87.2) | 64.3 (39.9-76.0) |
| **CA 19-9*, UI/L** | | | 2012 (0.8-636000) | 1399 (0.7-306000) |

| **Treatment Arms** | | | | |
|---|---|---|---|---|
| | **Arm A** | | N/A | 60 (33.9%) |
| | **Arm B** | | N/A | 64 (36.2%) |
| | **Arm C** | | N/A | 53 (29.9%) |
| **Detectable Met-DNA marker (one at least)** | | | 64 (64%) | 102 (57.6) |

### Statistical analysis

Statistical analyses were performed using SPSS software version 21.0 (SPSS Inc., Chicago, IL) and R Studio (*RStudio: Integrated Development for R. RStudio, Inc., Boston, MA). A P* value ≤ 0.05 was considered as significant. For the analysis of the hypermethylation difference between normal and adjacent tissues, Mann-whitney test was used.

### RESULTS:

*Identification of DNA methylation biomarkers based on TCGA analysis and validation.*

The results of the database analysis allowed us to select 10 CpG sites that met the established criteria (ie. DNA methylation level in tumor tissue, differences of DNA methylation level between tumor and normal tissues, correlation between the observed frequencies of DNA methylation and tumor cell content of the sample and the significance (p value) of the difference of DNA methylation level between tumor tissue and normal tissues). These CpG sites were located in the *POU4F1, HOXD8,* RYR2, XKR4, KCNA3 and PITX2 genes. *POU4F1* and *HOXD8* genes were selected to validate our procedure. *HOXD8* was selected because this gene contained 5 CpG sites among the 10 found. The *POU4F1* gene was selected because it contained the CpG site having the lowest mean methylation for DNA extracted from adjacent pancreatic tissues.

*Validation of DNA methylation changes of selected biomarkers by dPCR.*

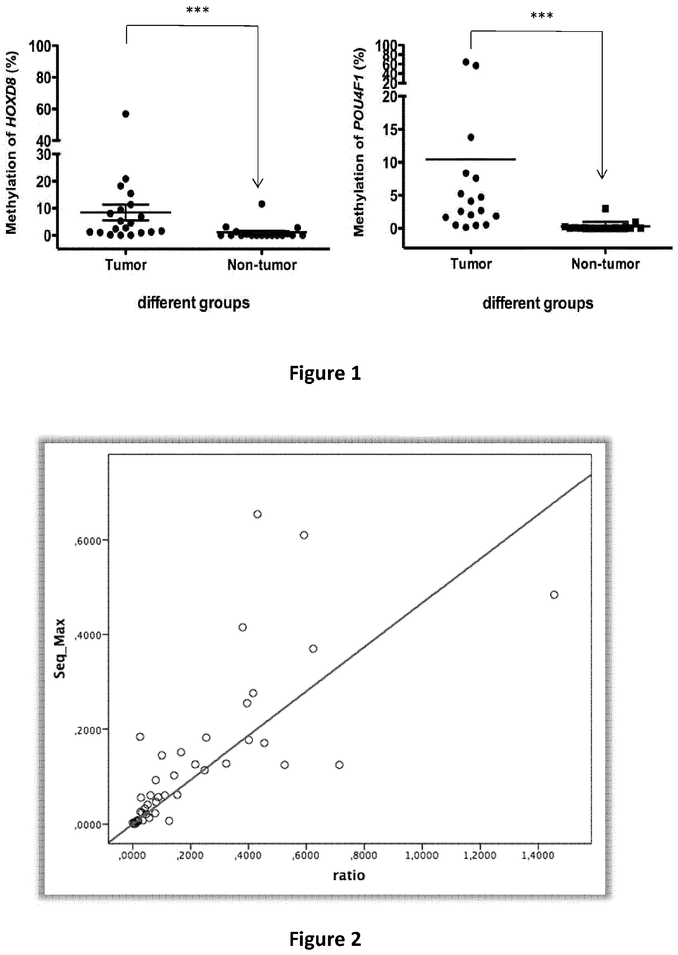

The difference of DNA methylation of several potential biomarkers in tumor and non-tumor tissue from pancreatic cancer patients was validated by ddPCR (n=20). Compared to those in non-tumor tissue, DNA methylation in tumor tissue was significantly increased. (Figure 1).

The detection sensitivity and specificity of the biomarkers *HOXD8 and POU4F1* alone by ddPCR in pancreatic cancers were more than 70% (Table 5):

**Table 5: Sensitivity and specificity of the biomarkers of the invention when assessed as single marker by ddPCR**

| | **HOXD8** | **POU4F1** |
|---|---|---|
| **Sensitivity** | **70%** *(14*/*20)* | **80%** *(16*/*20)* |
| **Specificity** | **89%** *(16*/*18)* | **94%** *(17*/*18)* |

### Combination of different biomarkers to reach a higher sensitivity and specificity

The sensitivity and specificity of each individual biomarker by ddPCR are shown in Table 5. The highest sensitivity with the use of only one biomarker is 80%. To reach a higher detection sensitivity and specificity, different biomarkers can be combined (Table 6). The detection sensitivity can furthermore be improved to 89% by combining *HOXD8 and POU4F1.*

**Table 6: Detection sensitivity and specificity of combination of selected biomarkers.**

| | **HOXD8 and POU4F1** |
|---|---|
| **Sensitivity** | **85%** |
| **Specificity** | **89%** |

### Methylation profiles of the selected biomarkers in plasma ccfDNA from healthy individuals by ddPCR

The purpose of these biomarkers is to detect DNA hypermethylation in pancreatic cancer patients but not in healthy individuals. In order to validate this, the methylation level was investigated in plasma ccfDNA from healthy individuals (n=12) by ddPCR. Among the tested markers, none were detected as positive with the combination of the two markers. One sample presented low positivity with one maker (*HOXD8,* 3 positive droplets (lower than LOD) corresponding to 0.4% of methylated DNA). Moreover, DNA extracted from whole Blood (commercial DNA from Promega) was also used to validate the assay and ensure that the markers were not positive in cells contained in the blood ensuring no false positive in case of blood cell hemolysis (for example due to pre-analytical sample handling).

### Comparison of ctDNA detection using the two biomarkers and using highly sensitive optimized NGS (BPER analysis).

64 samples previously analyzed by BPER-NGS from the Validation cohort were tested for the detection of ctDNA by the detection of methylation of the two gene candidates by dPCR. Among the 64 patients 20 appeared negative by both methods, 41 positives by both methods, 2 were positive only by dPCR methylation analysis and 1 by BPER-NGS only. It is important to note that this last result concerned a sample containing very low amount of ctDNA. The negativity is thus possibly due to the fact that bisulfite treatment could lead to loss of ctDNA and thus analysis of more DNA could have been pertinent here (and thus lead to positive results). However, it is clear from these results that the analyzes of the two candidate genes lead to results comparable to the one obtained using multiple gene mutation analyzes by targeted optimized NGS.

### Cross-table MUT_SEQ * METH_POS

**Table 7: Comparison between analyses of ctDNA by mutation detection using BPER NGS (MUT_SEQ) or Methylation analysis by the two candidate genes analysis (METH_POS). 0: negative samples, 1: positive samples.**

| Effectif | | | | |
|---|---|---|---|---|
| | | METH_POS | | Total |
| | | 0 | 1 | |
| MUT_SEQ | 0 | 20 | 2 | 22 |
| | 1 | 1 | 41 | 42 |
| Total | | 21 | 43 | 64 |

Finally, correlation has been also demonstrated between the frequencies of ctDNA determined by mutation-based BPER-NGS analysis (targeted sequencing) and methylation-based dPCR analysis (using our two marker genes) (Figure 2).

### Monitoring progression of pancreatic cancer.

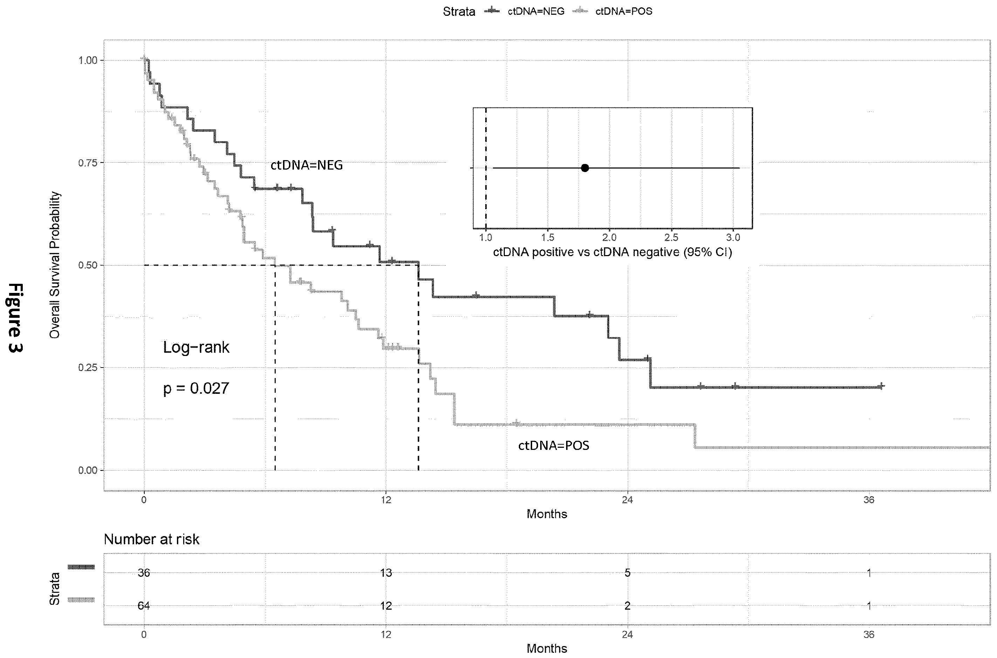

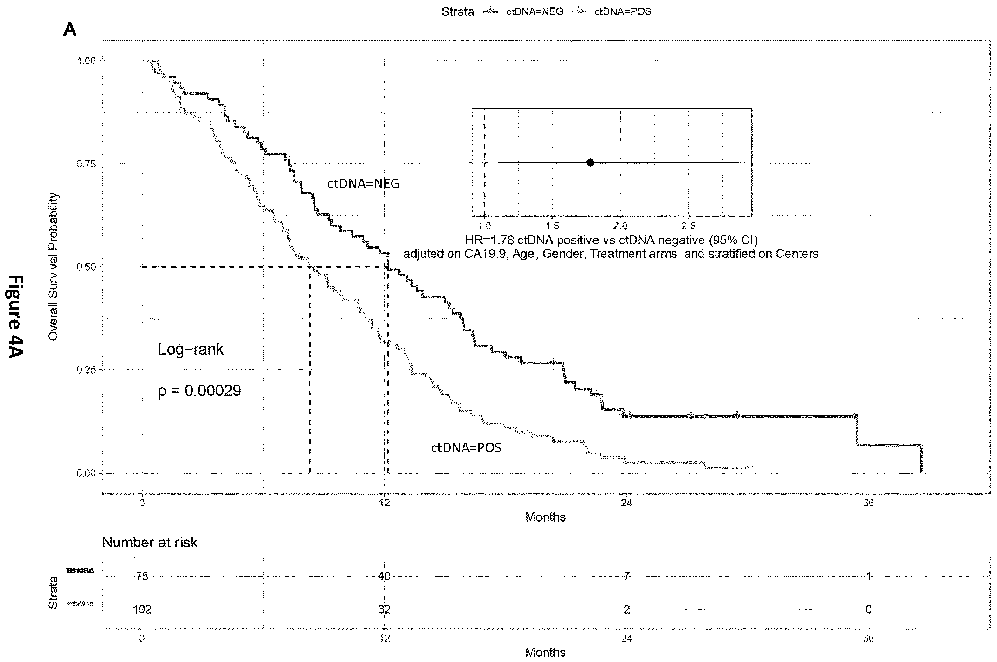

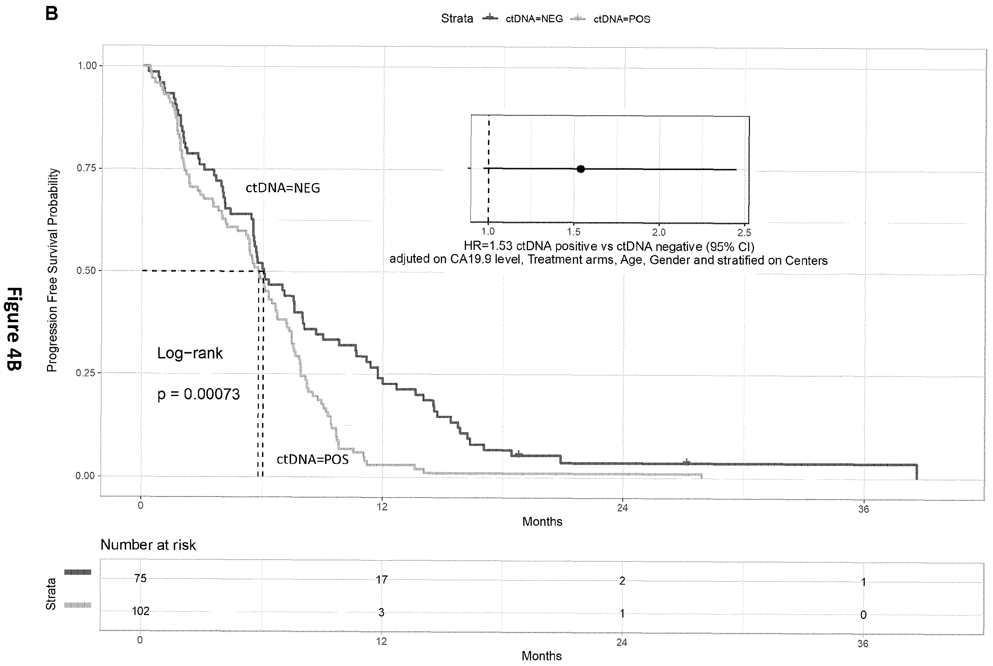

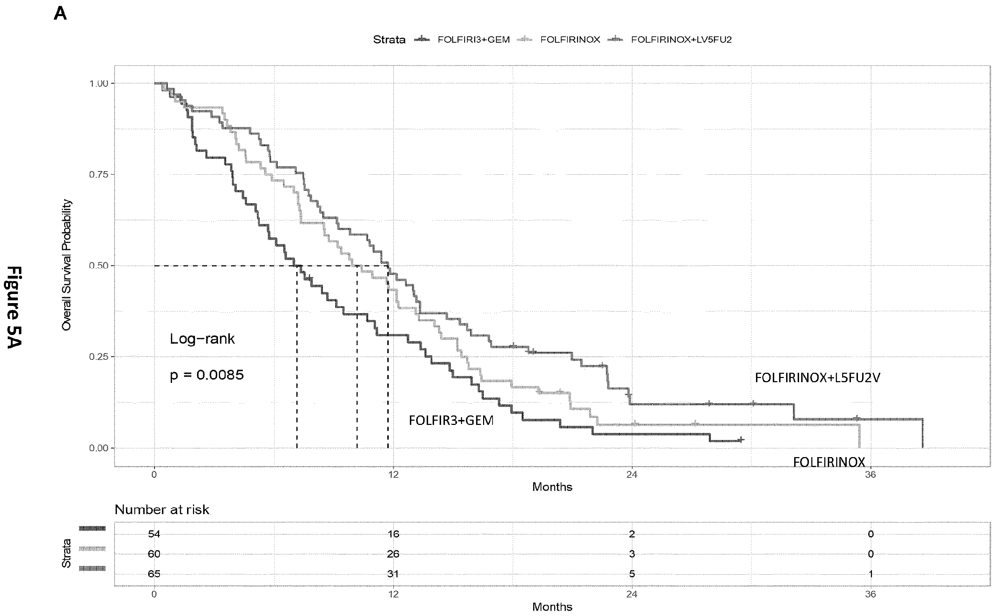

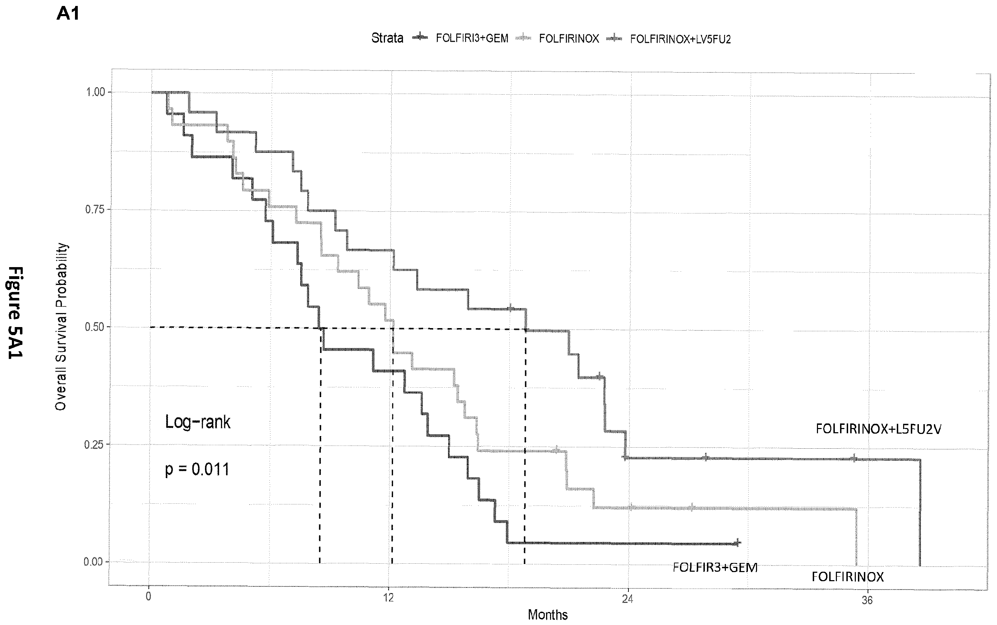

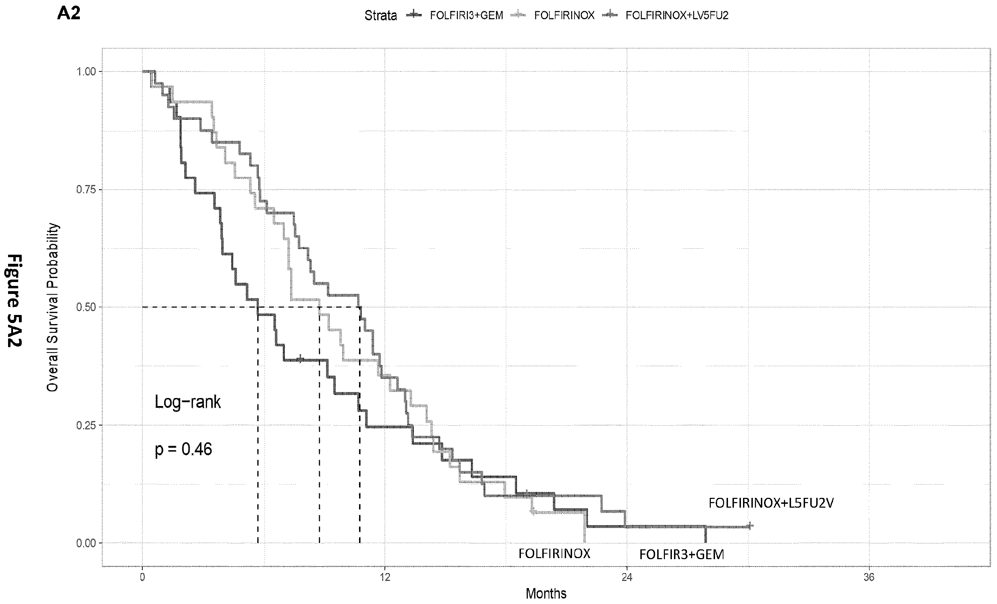

The methylation status of circulating cell free DNA as determined by the use of the two developed markers demonstrated to correlated with overall survival (Figure 3, Validation Cohort and Figure 4, Prodige 35 cohort). For the first cohort (Figure 3), 100 patients (Validation cohort) were tested for the presence of methylated tumor DNA by the above described procedure. Sixty-four percent (64%) were positive. An overall survival of 17.1 months was observed for patients negative for MetDNA (METH_POS=0) and 5.5 months for the patients positive for MetDNA (METH_POS=1). Similarly, for the Prodige 35 cohort, testing of the 177 patients demonstrated correlation between the presence of ctDNA, as detected by the methylation marker developed, and overall survival. Pertinence of these markers were further validated using univariate and multivariate analysis. In addition, in multivariate analysis adjusted on gender, age, CA19.9>40UI/mL, treatment arm, number of metastatic site and stratified on center, the Met-DNA was independently associated with poor OS in Prodige 35 cohort (see Table 8 and also Figure 5).

**Table 8. Univariate and multivariate analysis of the factors of overall survival in the Prodige 35 cohort.**

| | **Univariate Analysis** | | **Multivariate Analysis** | |
|---|---|---|---|---|
| | **Hazard ratio (IC 95%)** | ***P*** | **Hazard ratio (IC 95%)** | ***P*** |
| **OMS Status** | **1,51 (1,08 - 2,12)** | **0,015** | 1,56(1,10 - 2,12) | 0,012 |
| **CA 19-9** | **1,31 (1,06 - 1,62)** | **0,010** | 1,31 (1,06 - 1,61) | 0,011 |
| **Lymph/Platelet Ratio** | **0,788 (0,64 - 0,96)** | **0,024** | **1,30 (1,06 - 1,61)** | **0,172** |
| **Treatment arm** | 1,17 (0.94 - 1,46) | 0,576 | | |
| **Primitive surgery** | 0,65 (0,39 - 1,07) | 0,092 | | |
| **POS Methylation** | **1,42 (1,10 -1,82)** | **0,006** | 1,33 (1,01- 1,76) | 0,042 |

### Conclusion

These studies (Validation cohort and Prodige 35 cohort) demonstrate that Met-DNA is a strong independent prognostic marker in mPAC. These results argue for patient's stratification on ctDNA status for further randomized trials.

### REFERENCE

Bouvier AM, Remontet L, Jougla E, et al. Incidence of gastrointestinal cancers in France. Gastroenterologie clinique et biologique. 2004;28(10 Pt 1):877---881.
Coleman MP, Gatta G, Verdecchia A, et al. EUROCARE---3 summary: cancer survival in Europe at the end of the 20th century. Annals of oncology : official journal of the European Society for Medical Oncology / ESMO. 2003;14 Suppl 5:v128---149.
Eheman C, Henley SJ, Ballard---Barbash R, et al. Annual Report to the Nation on the status of cancer, 1975---2008, featuring cancers associated with excess weight and lack of sufficient physical activity. Cancer. 2012;118(9):2338---2366.
Burris HA, 3rd, Moore MJ, Andersen J, et al. Improvements in survival and clinical benefit with gemcitabine as first---line therapy for patients with advanced pancreas cancer: a randomized trial. Journal of clinical oncology: official journal of the American Society of Clinical Oncology. 1997;15(6):2403---2413.
Conroy T, Desseigne F, Ychou M, et al. FOLFIRINOX versus gemcitabine for metastatic pancreatic cancer. The New England journal of medicine. 2011;364(19):1817---1825.
Von Hoff DD, Ervin T, Arena FP, et al. Increased survival in pancreatic cancer with nabpaclitaxel plus gemcitabine. The New England journal of medicine. 2013;369(18):1691-1703.
Siegel RL, Miller KD, Jemal A. Cancer statistics, 2015. CA: a cancer journal for clinicians. 2015;65(1):5---29.
Bardeesy N, DePinho RA. Pancreatic cancer biology and genetics. Nature reviews. Cancer. 2002;2(12):897---909.
Almoguera C, Shibata D, Forrester K, Martin J, Arnheim N, Perucho M. Most human carcinomas of the exocrine pancreas contain mutant c---K---ras genes. Cell. 1988;53(4):549- --554.
Tada M, Omata M, Kawai S, et al. Detection of ras gene mutations in pancreatic juice and peripheral blood of patients with pancreatic adenocarcinoma. Cancer research. 1993;53(11):2472---2474. 41
Morris JPt, Wang SC, Hebrok M. KRAS, Hedgehog, Wnt and the twisted developmental biology of pancreatic ductal adenocarcinoma. Nature reviews. Cancer. 2010;10(10):683---695.
Kanda M, Matthaei H, Wu J, et al. Presence of somatic mutations in most early---stage pancreatic intraepithelial neoplasia. Gastroenterology. 2012;142(4):730---733 e739.
Hruban RH, Maitra A, Schulick R, et al. Emerging molecular biology of pancreatic cancer. Gastrointestinal cancer research : GCR. 2008;2(4 Suppl):S10---15.
Moore PS, Beghelli S, Zamboni G, Scarpa A. Genetic abnormalities in pancreatic cancer. Molecular cancer. 2003;2:7*.*
Poruk KE,Gay DZ, Brown K, Mulvihill JD, BoucherKM, Scaife CL, et al. The clinical utility of CA 19-9 in pancreatic adenocarcinoma: diagnostic and prognostic updates. Curr Mol Med 2013;13:340-51.
Dabritz J, Preston R, Hanfler J, Oettle H. Follow-up study of K-ras mutations in the plasma of patients with pancreatic cancer: correlation with clinical features and carbohydrate antigen 19-9. Pancreas 2009;38:534-41.
Mikeska T, et al. DNA methylation biomarkers in cancer: progress towards clinical implementation. Expert Rev Mol Diagn. 2012 Jun; 12(5):473-87.
Delpu Y et al, DNA methylation and cancer diagnosis. Int J Mol Sci. 2013 Jul 18;14(7):15029-58
Pecuchet et al. Clinical Chemistry 2016 & Pietrasz et al. Clinical Cancer Research 2017

## Claims

1. An *in vitro* method for diagnosing or identifying pancreas cancer in a subject, said method comprising determining the level or amount of methylation of the *POU4F1* gene or of the *HOXD8* gene in a body effluent of said subject.

2. The method according to claim 1, wherein if said gene is hypermethylated as compared with a reference sample, then said subject is diagnosed as suffering from a pancreas cancer.

3. The method according to any of claims 1 to 2, wherein said a body effluent is selected from urine, pancreatic juice, stools, preferably a blood sample.

4. The method according to any of claims 1 to 3, wherein said methylation is determined by Next Generation Sequencing (NGS) or by quantitative PCR (qPCR), preferably by digital PCR (dPCR).

5. The method according to any of claims 1 to 4, wherein said level or amount of methylation is determined in the nucleotide region of SEQ ID NO:1 in the *POU4F1* gene or in the nucleotide region of SEQ ID NO:3 in the *HOXD8* gene.

6. The method according to any of claims 1 to 5, wherein said level or amount of methylation of the *POU4F1* gene, or of the *HOXD8* gene is determined by dPCR by using the primers of SEQ ID NO:5 and SEQ ID NO:6 and/or SEQ ID NO:7 and SEQ ID NO:8 and using the probes of SEQ ID NO:9 and/or SEQ ID NO:10.

7. The method according to any of claims 1 to 6, comprising determining simultaneously or sequentially the level or amount of methylation of the *POU4F1* gene and the *HOXD8* gene in a body effluent of said subject.

8. An *in vitro* method for monitoring the evolution of pancreas cancer in a subject being diagnosed for pancreas cancer, said method comprising:
a) determining the level or amount of methylation of at least one gene selected from the group consisting of the *POU4F1* gene and the *HOXD8* gene in a body effluent of said subject, at a first time point,
b) determining the level or amount of methylation of said at least one gene selected previously in the step a), in a body effluent of said subject, at a second time point, and
c) comparing the level or amount of methylation determined in step b) to the level or amount determined in step a) or to a reference value.

9. The method according to claim 8, wherein the sample in step a) is obtained prior to the treatment for pancreas cancer and the sample in step b) is obtained after said subject has been treated for pancreas cancer.

10. The method according to claim 8, wherein the subject has been treated for pancreas cancer in step a) and the level or amount of methylation is determined once in step b) after said subject has been treated in step a) for pancreas cancer.

11. The method according to any one of claims 8 to 10, wherein the level or amount of methylation is determined simultaneously or sequentially in the *POU4F1* gene and in the *HOXD8* gene in a body effluent of said subject.

12. The method according to any one of claims 8 to 11, wherein said reference value is obtained in a healthy subject.

13. A method for determining or adapting a therapeutic regimen suitable for a subject diagnosed for pancreas cancer comprising the steps of:
a) determining the level or amount of methylation of *POU4F1* gene and/or *HOXD8* gene in a body effluent of the subject prior to administration of treatment or during treatment of said subject,
b) determining the level or amount of methylation of *POU4F1* gene and/or *HOXD8* gene in a body effluent of the subject after administration of treatment of said subject,
c) comparing the level or amount of methylation determined in step b) to the level or amount of methylation determined in step a), or to a reference value,
d) adapting/modifying the therapeutic regimen for the subject based on the comparison of step c).

14. A method for predicting a clinical outcome in a subject afflicted with pancreas cancer comprising the steps of:
a) determining the level or amount of methylation of *POU4F1* gene and/or *HOXD8* gene in a body effluent of said patient, and comparing said level or amount to a reference value,
b) predicting the clinical outcome based on the comparison of step a).

## Patentansprüche

1. In-vitro-Verfahren zum Diagnostizieren oder Identifizieren von Bauchspeicheldrüsenkrebs bei einem Subjekt, wobei dieses Verfahren das Bestimmen des Niveaus oder der Menge der Methylierung des POU4F1-Gens oder des *HOXD8*-Gens in einem Körperausfluss dieses Subjekts umfasst.

2. Das Verfahren nach Anspruch 1, wobei, wenn dieses Gen im Vergleich zu einer Referenzprobe hypermethyliert ist, bei dem Patienten Bauchspeicheldrüsenkrebs diagnostiziert wird.

3. Das Verfahren nach einem der Ansprüche 1 bis 2, wobei dieser Körperausfluss aus Urin, Pankreassaft, Stuhl, vorzugsweise einer Blutprobe, ausgewählt wird.

4. Das Verfahren nach einem der Ansprüche 1 bis 3, wobei die Methylierung durch Next Generation Sequencing (NGS) oder durch quantitative PCR (qPCR), vorzugsweise durch digitale PCR (dPCR), bestimmt wird.

5. Das Verfahren nach einem der Ansprüche 1 bis 4, wobei dieses Niveau oder diese Menge der Methylierung in der Nukleotidregion von SEQ ID NO:1 im *POU4F1-Gen* oder in der Nukleotidregion von SEQ ID NO:3 im *HOXD8*-Gen bestimmt wird.

6. Das Verfahren nach einem der Ansprüche 1 bis 5, wobei dieses Niveau oder diese Menge der Methylierung des POU4F1-Gens oder des *HOXD8*-Gens durch dPCR unter Verwendung der Primer von SEQ ID NO:5und SEQ ID NO:6 und/oder SEQ ID NO:7 und SEQ ID NO:8und unter Verwendung der Sonden von SEQ ID NO:9 und/oder SEQ ID NO:10 bestimmt wird.

7. Das Verfahren nach einem der Ansprüche 1 bis 6, umfassend das gleichzeitige oder sequentielle Bestimmen des Niveaus oder der Menge der Methylierung des POU4F1-Gens und des HOXD8-Gens in einem Körperausfluss des Subjekts.

8. *In-vitro-*Verfahren zur Überwachung der Entwicklung von Bauchspeicheldrüsenkrebs bei einem Subjekt, bei dem Bauchspeicheldrüsenkrebs diagnostiziert wurde, wobei dieses Verfahren Folgendes umfasst:
*a)* Bestimmen des Niveaus oder der Menge der Methylierung von mindestens einem Gen, das aus der Gruppe ausgewählt ist, die aus dem POU4F1-Gen und dem HOXD8-Gen besteht, in einem Körperausfluss dieses Subjekts zu einem ersten Zeitpunkt,
*b)* Bestimmen des Niveaus oder der Menge der Methylierung von mindestens einem Gen, das aus der Gruppe ausgewählt ist, die aus dem POU4F1-Gen und dem HOXD8-Gen besteht, in einem Körperausfluss dieses Subjekts zu einem zweiten Zeitpunkt, und
*c)* Vergleichen des Niveaus oder der bestimmten Menge der Methylierung, bestimmt in Schritt b) mit dem Niveau oder der Menge, bestimmt in Schritt a), oder mit einem Referenzwert.

9. Das Verfahren nach Anspruch 8, wobei die Probe in Schritt a) vor der Behandlung gegen Bauchspeicheldrüsenkrebs gewonnen wird und die Probe in Schritt b) nach der Behandlung dieses Subjekts gegen Bauchspeicheldrüsenkrebs gewonnen wird

10. Das Verfahren nach Anspruch 8, wobei das Subjekt in Schritt a) gegen Bauchspeicheldrüsenkrebs behandelt wurde und das Niveau oder die Menge der Methylierung einmal in Schritt b) bestimmt wird, nachdem das Subjekt in Schritt a) gegen Bauchspeicheldrüsenkrebs behandelt wurde.

11. Das Verfahren nach einem der Ansprüche 8 bis 10, wobei das Niveau oder die Menge der Methylierung gleichzeitig oder sequentiell im *POU4F1-Gen* und im *HOXD8*-Gen in einem Körperausfluss dieses Subjekts bestimmt wird.

12. Das Verfahren nach einem der Ansprüche 8 bis 11, wobei dieser Referenzwert bei einem gesunden Probanden erhalten wird.

13. Verfahren zum Bestimmen oder Anpassen eines geeigneten Therapieschemas für ein Subjekt, bei dem Bauchspeicheldrüsenkrebs diagnostiziert wurde, umfassend die Schritte:
a) Bestimmen des Niveaus oder der Menge der Methylierung des POU4F1-Gens und/oder des HOXD8-Gens in einem Körperausfluss des Subjekts vor Verabreichung der Behandlung oder während der Behandlung dieses Subjekts,
*b)* Bestimmen des Niveaus oder der Menge der Methylierung des POU4F1-Gens und/oder des HOXD8-Gens in einem Körperausfluss des Subjekts nach Verabreichung der Behandlung des Subjekts,
*c)* Vergleichen des Niveaus oder der Menge der Methylierung bestimmt in Schritt b) mit dem Niveau oder der Menge der Methylierung bestimmt in Schritt a) oder mit einem Referenzwert,
*d)* Anpassen/Ändern des Therapieschemas für das Subjekt basierend auf dem Vergleich aus Schritt c).

14. Verfahren zur Vorhersage eines klinischen Ergebnisses bei einem Subjekt mit Bauchspeicheldrüsenkrebs, umfassend die Schritte:
*a)* Bestimmen des Niveaus oder der Menge der Methylierung des POU4F1-Gens und/oder des HOXD8-Gens in einem Körperausfluss dieses Patienten und Vergleichen dieses Niveaus oder dieser Menge mit einem Referenzwert,
*b)* Vorhersage des klinischen Ergebnisses basierend auf dem Vergleich aus Schritt a).

## Revendications

1. Méthode *in vitro* pour diagnostiquer ou identifier un cancer du pancréas chez un sujet, ladite méthode comprenant la détermination du niveau ou de la quantité de méthylation du gène *POU4F1* ou du gène *HOXD8* dans un effluent corporel dudit sujet.

2. Méthode selon la revendication 1, dans laquelle, si ledit gène est hyperméthylé en comparaison avec un échantillon de référence, alors ledit sujet est diagnostiqué comme souffrant d'un cancer du pancréas.

3. Méthode selon l'une quelconque des revendications 1 et 2, dans laquelle ledit effluent corporel est choisi parmi l'urine, le suc pancréatique, les selles, de préférence un échantillon de sang.

4. Méthode selon l'une quelconque des revendications 1 à 3, dans laquelle ladite méthylation est déterminée par séquençage de nouvelle génération (NGS) ou par PCR quantitative (qPCR), de préférence par PCR digitale (dPCR).

5. Méthode selon l'une quelconque des revendications 1 à 4, dans laquelle ledit niveau ou quantité de méthylation est déterminé dans la région de nucléotides de la SEQ ID NO : 1 dans le gène *POU4F1* ou dans la région de nucléotides de la SEQ ID NO : 3 dans le gène *HOXD8.*

6. Méthode selon l'une quelconque des revendications 1 à 5, dans laquelle ledit niveau ou quantité de méthylation du gène *POU4F1,* ou du gène *HOXD8,* est déterminé par dPCR par utilisation des amorces de la SEQ ID NO : 5 et de la SEQ ID NO : 6 et/ou de la SEQ ID NO : 7 et de la SEQ ID NO : 8 et par utilisation des sondes de la SEQ ID NO : 9 et/ou de la SEQ ID NO : 10.

7. Méthode selon l'une quelconque des revendications 1 à 6, comprenant la détermination simultanée ou successive du niveau ou de la quantité de méthylation du gène *POU4F1* et du gène *HOXD8* dans un effluent corporel dudit sujet.

8. Méthode *in vitro* pour surveiller l'évolution d'un cancer du pancréas chez un sujet diagnostiqué avec un cancer du pancréas, ladite méthode comprenant :
a) la détermination du niveau ou de la quantité de méthylation d'au moins un gène choisi dans le groupe constitué par le gène *POU4F1* et le gène *HOXD8* dans un effluent corporel dudit sujet, à un premier point de temps,
b) la détermination du niveau ou de la quantité de méthylation dudit au moins un gène sélectionné antérieurement dans l'étape a), dans un effluent corporel dudit sujet, à un deuxième point de temps, et
c) la comparaison du niveau ou de la quantité de méthylation déterminé dans l'étape b) au niveau ou à la quantité déterminé dans l'étape a) ou à une valeur de référence.

9. Méthode selon la revendication 8, dans laquelle l'échantillon dans l'étape a) est obtenu avant le traitement pour un cancer du pancréas et l'échantillon dans l'étape b) est obtenu après que ledit sujet a été traité pour un cancer du pancréas.

10. Méthode selon la revendication 8, dans laquelle le sujet a été traité pour un cancer du pancréas dans l'étape a) et le niveau ou la quantité de méthylation est déterminé une fois dans l'étape b) après que ledit sujet a été traité dans l'étape a) pour un cancer du pancréas.

11. Méthode selon l'une quelconque des revendications 8 à 10, dans laquelle le niveau ou la quantité de méthylation est déterminé simultanément ou successivement dans le gène *POU4F1* et dans le gène *HOXD8* dans un effluent corporel dudit sujet.

12. Méthode selon l'une quelconque des revendications 8 à 11, dans laquelle ladite valeur de référence est obtenue chez un sujet en bonne santé.

13. Méthode pour déterminer ou adapter un régime thérapeutique convenant pour un sujet diagnostiqué avec un cancer du pancréas, comprenant les étapes de :
a) détermination du niveau ou de la quantité de méthylation du gène *POU4F1* et/ou du gène *HOXD8* dans un effluent corporel du sujet avant l'administration du traitement ou durant le traitement dudit sujet,
b) détermination du niveau ou de la quantité de méthylation du gène *POU4F1* et/ou du gène *HOXD8* dans un effluent corporel du sujet après l'administration du traitement audit sujet,
c) comparaison du niveau ou de la quantité de méthylation déterminé dans l'étape b) au niveau ou à la quantité de méthylation déterminé dans l'étape a), ou à une valeur de référence,
d) adaptation/modification du régime thérapeutique pour le sujet sur la base de la comparaison de l'étape c).

14. Méthode pour prédire un résultat clinique chez un sujet affligé d'un cancer du pancréas, comprenant les étapes de :
a) détermination du niveau ou de la quantité de méthylation du gène *POU4F1* et/ou du gène *HOXD8* dans un effluent corporel dudit patient, et comparaison dudit niveau ou quantité à une valeur de référence,
b) prédiction du résultat clinique sur la base de la comparaison de l'étape a).
